(19) Europäisches Patentamt
European Patent Office
Office européen des brevets

(11) EP 3 847 146 B1

(12) EUROPÄISCHE PATENTSCHRIFT

(45) Veröffentlichungstag und Bekanntmachung des Hinweises auf die Patenterteilung:
**11.10.2023 Patentblatt 2023/41**

(21) Anmeldenummer: **19756205.1**

(22) Anmeldetag: **26.08.2019**

(51) Internationale Patentklassifikation (IPC):
***C07C 29/151*** (2006.01) ***C07C 29/78*** (2006.01)
***C07C 29/80*** (2006.01)

(52) Gemeinsame Patentklassifikation (CPC):
(C-Sets verfügbar)
**C01B 3/382; C07C 29/1518; C07C 29/78; C07C 29/80;** C01B 2203/0244; C01B 2203/0261; C01B 2203/0415; C01B 2203/043; C01B 2203/0475; C01B 2203/061; C01B 2203/148; Y02P 20/129 (Forts.)

(86) Internationale Anmeldenummer:
**PCT/EP2019/072713**

(87) Internationale Veröffentlichungsnummer:
**WO 2020/048809 (12.03.2020 Gazette 2020/11)**

(54) **VERFAHREN ZUR HERSTELLUNG VON METHANOL AUS SYNTHESEGAS OHNE EMISSION VON KOHLENDIOXID**

METHOD FOR PRODUCING METHANOL FROM SYNTHESIS GAS WITHOUT THE EMISSION OF CARBON DIOXIDE

PROCÉDÉ DE PRODUCTION DE MÉTHANOL À PARTIR DE GAZ DE SYNTHÈSE SANS ÉMISSION DE DIOXYDE DE CARBONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.09.2018 EP 18192465**

(43) Veröffentlichungstag der Anmeldung:
**14.07.2021 Patentblatt 2021/28**

(73) Patentinhaber: **BASF SE**
**67056 Ludwigshafen am Rhein (DE)**

(72) Erfinder:
- **VICARI, Maximilian**
  **67056 Ludwigshafen (DE)**
- **GEIGER, Thomas**
  **67056 Ludwigshafen (DE)**
- **KATZ, Torsten**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
**EP-A1- 2 228 358** **EP-A1- 2 751 307**
**EP-A1- 3 178 804**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



(Forts. nächste Seite)

(52) Gemeinsame Patentklassifikation (CPC): (Forts.)

C-Sets
**C07C 29/1518, C07C 31/04;**
**C07C 29/78, C07C 31/04;**
**C07C 29/80, C07C 31/04**

## Beschreibung

Die vorliegende Erfindung betrifft ein Verfahren zur Herstellung von Methanol aus Synthesegas, bei dem die kohlenstoffhaltigen Verbindungen der in der Isolierung des Methanols abgetrennten Ströme zu Kohlendioxid umgewandelt und unter Vermeidung von dessen Emission erneut in der Herstellung von Methanol eingesetzt werden. Das Verfahren basiert dabei auf der, dem Fachmann bekannten, kontinuierlich betriebenen Methanolsynthese nach dem Niederdruckverfahren.

Methanol ist einer der bedeutendsten Synthese-Rohstoffe weltweit und wird neben dessen Einsatz als Lösungsmittel unter anderem für die Synthesen von Formaldehyd, Essigsäure, Methyltert-butylether (MTBE), Dimethylterephthalat, Methylmethacrylat und Methylaminen in großen Mengen eingesetzt.

Methanol wird großtechnisch aus Synthesegas in einem Reaktor in Gegenwart eines Methanol-Synthesekatalysators erzeugt. Das Synthesegas enthält hauptsächlich Wasserstoff und Kohlenmonoxid, sowie abhängig von der Art der Herstellung und Aufarbeitung auch entsprechende Mengen an Kohlendioxid, Wasser und sogenannte Inertgase wie etwa Methan, Stickstoff oder Argon.

Gemäß Ullmann's Encyclopedia of Industrial Chemistry, Kapitel "Methanol", 2012 Wiley-VCH Verlag GmbH & Co. KGaA, Weinheim, Germany, erfolgt die Umsetzung von Synthesegas zu Methanol üblicherweise im sogenannten Niederdruckverfahren in einem Druckbereich von 5 bis 10 MPa abs an Kupfer und Zink enthaltenden Methanol-Synthesekatalysatoren. Dabei wird sowohl Kohlenmonoxid als auch Kohlendioxid zu Methanol umgesetzt.

$$CO + 2\,H_2 \rightleftharpoons CH_3OH \quad (1)$$

$$CO_2 + 3\,H_2 \rightleftharpoons CH_3OH + H_2O \quad (2)$$

Basierend auf den Reaktionsgleichungen (1) und (2) ergibt sich als Stöchiometriezahl S für die Methanolsynthese

$$S = \frac{n(H_2) - n(CO_2)}{n(CO) + n(CO_2)} \quad (3)$$

wobei n jeweils für die jeweiligen Molmengen steht. Eine Stöchiometriezahl S von 2 entspricht dabei der rechnerischen Stöchiometrie. Da jedoch ein Defizit an Wasserstoff die Selektivität zu Methanol stark reduziert, wird eine Stöchiometriezahl S von geringfügig über 2 als Optimum für die Methanolsynthese gesehen.

Das für die Methanolsynthese einzusetzende Synthesegas wird dabei üblicherweise aus Erdgas, anderen Kohlenwasserstoffe enthaltenden Strömen, sowie teilweise auch durch Kohlevergasung oder Holzvergasung gewonnen. Als gängige Herstellverfahren für Synthesegas nennt Ullmann's Encyclopedia of Industrial Chemistry, Kapitel "Methanol", 2012 Wiley-VCH Verlag GmbH & Co. KGaA, Weinheim, Germany folgende vier Verfahren.

a) Die Dampfreformierung, bei der ein Kohlenwasserstoff-Feedgas unter Zusatz von Wasserdampf und Abwesenheit von Sauerstoff katalytisch in Wasserstoff, Kohlenmonoxid und Kohlendioxid endotherm gespalten wird.

b) Die autotherme Reformierung, bei der ein Kohlenwasserstoff-Feedgas in Anwesenheit eines nickelhaltigen Katalysators mit Sauerstoff partiell zu Wasserstoff, Kohlenmonoxid und Kohlendioxid exotherm oxidiert wird.

c) Die Kombination aus der Dampfreformierung und der autothermen Reformierung.

d) Die partielle Oxidation, bei der ein Kohlenwasserstoff-Feedgas in Abwesenheit eines Katalysators mit Sauerstoff partiell zu Wasserstoff, Kohlenmonoxid und Kohlendioxid exotherm oxidiert wird. Durch die Abwesenheit eines Katalysators weist die partielle Oxidation gegenüber der autothermen Reformierung jedoch praktische Nachteile auf.

Bei der Dampfreformierung wird in der Regel ein Synthesegas mit einer Stöchiometriezahl S von etwa 3 erzeugt. Hinsichtlich der Methanolsynthese liegt somit ein deutlicher Wasserstoff-Überschuss vor. Bei der autothermen Reformierung und der partiellen Oxidation wird hingegen ein Synthesegas mit einer Stöchiometriezahl S von $< 2$ gebildet. Daher bietet insbesondere die Kombination aus der Dampfreformierung und der autothermen Reformierung eine technisch verbreitete Variante, welche es gestattet, die Stöchiometriezahl S auf einen technisch relevanten Wert von $> 2$ gezielt einzustellen.

Da bei der Synthesegaserzeugung das Kohlenwasserstoff-Feedgas üblicherweise nicht vollständig umgesetzt

wird, enthält das Synthesegas in der Regel mehr oder weniger große Anteile an nicht umgesetzten Kohlenwasserstoffen, wie beispielsweise Methan. Zudem werden beim Einsatz von Luft als Oxidator bei der Synthesegaserzeugung auch mehr oder weniger große Anteile an inerten Fremdgasen, wie beispielsweise Stickstoff oder Argon eingebracht. Diese werden in der Regel nicht gesondert abgetrennt, sondern zusammen mit dem werthaltigen Wasserstoff, Kohlenmonoxid und Kohlendioxid der Methanolsynthese zugeführt.

Auch wenn es sich bei den Reaktionsgleichungen (1) und (2) um Gleichgewichtsgleichungen handelt und Methanol-Synthesekatalysator zugegen ist, stellt sich das Gleichgewicht nicht quantitativ ein. Daher enthält das Reaktionsgemisch direkt nach der Methanolsynthese üblicherweise nur etwa 5 bis 15 Gew.-% Methanol und nicht unerhebliche Mengen an nicht umgesetztem Wasserstoff, Kohlenmonoxid, Kohlendioxid, sowie Fremdgase wie Methan, Stickstoff oder Argon. Zudem enthält das Reaktionsgemisch auch Nebenprodukte wie beispielsweise Dimethylether. Im Stand der Technik sind verschiedene Verfahren zur Aufarbeitung des erhaltenen Reaktionsgemischs beschrieben. Zentrale Schritte sind üblicherweise die stufenweise Aufkonzentrierung des Methanols, ein möglichst effizienter Umgang mit den werthaltigen Gasen, und die Vermeidung einer Aufpegelung von Fremdgasen.

So wird bei dem in Ullmann's Encyclopedia of Industrial Chemistry, Kapitel "Methanol", Kapitel 5.2.2, 2012 Wiley-VCH Verlag GmbH & Co. KGaA, Weinheim, Germany, beschriebenen Lurgi MegaMethanol-Verfahren Synthesegas in einem Methanol-Synthesereaktor heterogenkatalytisch zu Methanol umgesetzt und aus dem erhaltenen Reaktionsgemisch zunächst ein mit Methanol angereicherter Roh-Methanolstrom auskondensiert. Der verbleibende Gasstrom enthält unter anderem nicht umgesetzten Wasserstoff und wird zu dessen Rückgewinnung einer Druckwechseladsorption zugeführt. Der so rückgewonnene Wasserstoff wird zur Methanolsynthese rückgeführt. Der in der Druckwechseladsorption nicht absorbierte Gasstrom wird schließlich der thermischen Verwertung zugeführt. Der durch die oben genannte Auskondensation erhaltene und mit Methanol angereicherte Flüssigkeitsstrom wird anschließend zur Ausgasung entspannt und das Entspannungsgas ebenfalls der thermischen Verwertung zugeführt. Der nach der Ausgasung verbleibende, mit Methanol weiter angereicherte Flüssigkeitsstrom wird dann zur eigentlichen Methanolgewinnung einer mehrstufigen Destillation unterzogen. Dabei wird auch der Abgasstrom der Leichtsiederkolonne der thermischen Verwertung zugeführt.

Bei dem beschriebenen Lurgi MegaMethanol-Verfahren wird zwar nicht umgesetzter Wasserstoff abgetrennt und zur Synthese rückgeführt, jedoch sämtliche übrigen werthaltigen Gase nach deren Abtrennung lediglich einer thermischen Verwertung zugeführt. Somit bleiben auch das nicht umgesetzte Kohlenmonoxid und Kohlendioxid für die weitere Methanolgewinnung ungenutzt. Auch wenn diese für die weitere Methanolgewinnung ungenutzt bleiben, sind deren Mengenströme jedoch bei der Auslegung der Anlage zu berücksichtigen, etwa in Form der erforderlichen Apparatedimensionen zum Handling der Mengenströme. Des Weiteren benötigt das Handling dieser Mengenströme natürlich auch Energie, etwa in Form von Heiz-, Kompressions- oder Pumpenergie, ohne dass diese zur weiteren Methanolsynthese genutzt werden. Zudem führt die thermische Verwertung der werthaltigen Gase zu einer weiteren Zunahme der Kohlendioxid-Emission. Brennbare Gase, zu denen auch Kohlenmonoxid gehört, werden zu Kohlendioxid umgesetzt; das bereits vorhandene Kohlendioxid selbst wird in der thermischen Verwertung unverändert hindurchgeleitet.

EP 3,178,804 A1 offenbart ein Methanol-Syntheseverfahren aus Synthesegas, bei dem das bei der Methanol-Synthese nicht umgesetzte Synthesegas nach der Abtrennung des Methanols zur Gewinnung thermischer Energie für die Wärmeversorgung des Synthesegas-Reformer genutzt wird. Bei dem beschriebenen Verfahren wird das abgetrennte, nicht umgesetzte Synthesegas zusammen mit Erdgas in einer Verbrennungseinheit am Synthesegas-Reformer verbrannt und somit ein kleiner Teil des Erdgases eingespart, was wiederum zu einer Verringerung der Kohlendioxid-Emission im Gesamtverfahren führt.

In EP 2,228,358 A1 wurde erkannt, dass auch das im Roh-Methanol nach dessen Synthese aus dem Synthesegas enthaltene Kohlendioxid einen wertvollen Einsatzstoff für die weitere Methanolsynthese darstellt. Dies gilt insbesondere für die Methanolsynthese, bei der das Synthesegas durch Dampfreformierung gewonnen wurde, da hier aufgrund einer Stöchiometriezahl S von etwa 3 ein deutlicher Überschuss an Wasserstoff vorliegt. EP 2,228,358 A1 lehrt, kohlendioxidhaltige Ströme aus der Methanolsynthese ohne weitere Aufarbeitung als Einsatzgasstrom zur Gewinnung von Synthesegas zum Reformer zurückzuführen und somit das Kohlendioxid erneut als Kohlenstoffquelle für die Methanolsynthese zu nutzen. Abbildung 1 in EP 2,228,358 A1 offenbart konkret die Rückführung des Entspannungsgases "9" aus der Entspannung des Methanol-Reaktoraustrags sowie des Abgasstroms "10" der Leichtsiederkolonne als zusätzlicher Einsatzgasstrom zum Reformer. Das Abgas "18" aus der Wasserstoff-Rückgewinnung wird jedoch dem Reformer als Brenngas zugeführt. EP 2,228,358 A1 lehrt zudem, auch das im Rauchgas des Reformers enthaltende Kohlendioxid als Strom "11" über eine Kohlendioxid-Rückgewinnungseinheit abzutrennen und dem Reformer als Einsatzgasstrom und somit zur weiteren Nutzung in der nachfolgenden Methanolsynthese zuzuführen.

Nachteilig an dem beschriebenen Verfahren ist vor allem, dass durch die direkte Rückführung des Entspannungsgases und des Abgasstroms der Leichtsiederkolonne auch die Inertgase wie etwa Stickstoff und Argon rückgeführt werden, und sich diese somit im Verfahren anreichern. Auch wenn der Abgasstrom der Wasserstoff-Rückgewinnung als Brenngas zum Reformer rückgeführt wird und dadurch eine gewisse Ausschleusung der Inertgase bewirkt wird, steigt dennoch die Kreisgasmenge an und der auf die Kreisgasmenge bezogene Umsatz zu Methanol nimmt ab. Zudem

ist auch der Reformer auf die zusätzliche Gasmenge infolge der Rückführung entsprechend groß auszulegen. Die Kohlendioxid-Rückgewinnung aus dem Rauchgas des Reformers erfordert zudem eine entsprechend groß dimensionierte Gaswäscheeinheit einschließlich deren energetische Einbindung in den Gesamtkomplex.

Auch US 8,829,059 befasst sich mit der Rückgewinnung und Wiederverwendung von Kohlendioxid aus der Methanolsynthese. Konkret lehrt die Schrift ein Methanol-Syntheseverfahren, bei dem das Reaktionsgemisch aus dem Methanol-Synthesereaktor zur Auskondensierung von wasserhaltigen Roh-Methanol einem Kondensator zugeführt wird und das verbleibende, nicht auskondensierte Gas in zwei Teilströme aufgesplittet wird. Ein Teilstrom wird dabei über die Permeat-Seite einer Kohlendioxid-sensitiven Membraneinheit geleitet und dann weiter über den Synthesegas-Kompressor zurück zum Methanol-Synthesereaktor geführt. Der andere Teilstrom wird zur Retentat-Seite einer Wasserstoff-sensitiven Membraneinheit geleitet, in der auf der Permeat-Seite ein mit Wasserstoff angereicherter Strom zur Wiederverwendung in der Methanolsynthese erzeugt wird. Der an Wasserstoff abgereicherte Strom der Retentat-Seite wird dann zur Retentat-Seite der bereits genannten Kohlendioxid-sensitiven Membraneinheit geleitet, um dort den Kohlendioxid-Gehalt auf der Permeat-Seite zu erhöhen und somit weiteres Kohlendioxid zur Methanolsynthese zurückzuführen. Das beschriebene Verfahren ist in Abbildung 3 der US 8,829,059 in einem Blockdiagramm dargestellt.

Der Einsatz von Membraneinheiten in Anlagen von der Größe einer technischen Methanol-Syntheseanlage ist relativ aufwändig, da für das Handling der großen Gasströme auch entsprechend große Membranflächen erforderlich sind. Dies gilt vor allem, wenn ein hoher Trennungsgrad erreicht werde soll. Umgekehrt würde jedoch bei einem niedrigen Trennungsgrad ein Teil des Wasserstoffs und Kohlendioxids jeweils auf der Retentat-Seite verbleiben und ungenutzt aus dem Methanol-Syntheseverfahren ausgeschleust werden. Des Weiteren besteht das Risiko, dass die Membrane mit der Zeit durch Ablagerungen aus diversen Verunreinigungen und/oder Nebenprodukten der Methanolsynthese an Durchlässigkeit verlieren und von Zeit zu Zeit ausgetauscht oder aufwändig gereinigt werden müssen. Zudem schweigt sich US 8,829,059 über die weitere Aufarbeitung des im Kondensator auskondensierten wasser- und methanolhaltigen Stroms aus, welcher typischerweise noch erhebliche Mengen an in Methanol und Wasser gelöstem Kohlendioxid enthält.

S. Reddy et al. in Energy Procedia 63 (2014) 1407-1414 schlägt ebenfalls vor, Kohlendioxid aus der Synthesegas-Erzeugung beziehungsweise aus kohlendioxidhaltigen Strömen der Methanol-synthese zur weiteren Methanolsynthese zu nutzen. Konkret lehrt die Publikation, das Kohlendioxid aus dem Rauchgas des Synthesegasreformers durch eine "Econamine FG Plus$^{SM}$" Gaswäscheeinheit in einem aminischen Lösungsmittel zu absorbieren, anschließend als Kohlendioxidstrom wieder freizusetzen und diesen Kohlendioxidstrom dann entweder zusammen mit dem Einsatzgasstrom dem Reformer zur Erzeugung des Synthesegases oder gleich direkt dem Synthesegas wieder zuzuführen. Zudem lehrt die Publikation, sogenannte Purge-Gase und Abgase aus der Aufarbeitung des Methanolstroms direkt zur Kompressionsstufe vor dem Methanol-Synthesereaktor zurückzuführen und/oder dem Reformer als Brenngas zuzuführen. In beiden Fällen wird das darin enthaltende Kohlendioxid erneut der Methanolsynthese zugeführt. Durch die beschriebenen Maßnahmen soll eine Methanol-Kapazitätserhöhung von etwa 20% möglich sein.

Auch bei diesem Verfahren ist die direkte Rückführung von Purge-Gasen und Abgasen zum Methanol-Synthesereaktor nachteilig, da sich hierdurch Inertgase wie etwa Stickstoff und Argon, aber auch Methan oder Nebenprodukte wie Dimethylether im Synthesekreiskauf anreichern. In der Variante, in der die genannten Gasströme dem Brenngas zum Reformer zugeführt werden, wird zwar einer Aufpegelung von Inertgasen und Nebenprodukten entgegengewirkt, aber durch die zusätzliche Gasmenge ist der Reformer entsprechend groß auszulegen. Die Kohlendioxid-Rückgewinnung aus dem Rauchgas des Reformers erfordert zudem eine entsprechend groß dimensionierte Gaswäscheeinheit einschließlich deren energetische Einbindung in den Gesamtkomplex.

Aufgabe der vorliegenden Erfindung war es, ein Verfahren zur Herstellung von Methanol aus Synthesegas zu finden, welches die oben genannten Nachteile nicht oder in nur untergeordnetem Maße aufweist und das im Synthesegas enthaltene Kohlenmonoxid und Kohlendioxid nahezu vollständig mit Wasserstoff zu Methanol umsetzt und somit, bezogen auf das Synthesegas, Kohlendioxid-emissionsfrei arbeitet. Das erfindungsgemäße Verfahren soll zudem einfach durchzuführen sein und weitgehend auf die technisch üblichen Apparate und Verschaltungen etablierter Methanol-Syntheseverfahren zurückgreifen. Somit soll das erfindungsgemäße Verfahren auch eine leichte Nachrüstung bestehender Methanol-Syntheseverfahren ermöglichen. Technische Eingriffe, Umbauten oder Ergänzungen bei der Synthesegaserzeugung sollen der Einfachheit halber möglichst vermieden werden.

Es wurde ein Verfahren zur Herstellung von Methanol gefunden, bei dem man

(a) aus einem kohlenstoffhaltigen Einsatzstoff (I) in einer Synthesegas-Erzeugungseinheit (A) ein Kohlenmonoxid, Kohlendioxid und Wasserstoff enthaltendes Synthesegas (II) erzeugt,

(b) das Synthesegas (II) aus Stufe (a) einer Methanol-Syntheseeinheit (B) zuführt und bei einer Temperatur von 150 bis 300°C und einem Druck von 5 bis 10 MPa abs in Gegenwart eines Methanol-Synthesekatalysators zu einem Methanol, Wasser, Kohlenmonoxid, Kohlendioxid, Wasserstoff, Dimethylether und Methan haltigen Reaktionsgemisch umsetzt, aus diesem einen mit Methanol und Wasser angereicherten Roh-Methanolstrom (III) auskondensiert, und den Roh-Methanolstrom (III) sowie einen Kohlenmonoxid, Kohlendioxid, Wasserstoff und Methan enthaltenden

gasförmigen Strom (IV) aus der Methanol-Syntheseeinheit (B) abführt,

(c) den Roh-Methanolstrom (III) aus Stufe (b) in einer Entspannungseinheit (C) auf einen Druck von 0,1 bis 2 MPa abs entspannt und ein Kohlendioxid und Methan enthaltendes Entspannungsgas (V) sowie einen mit Methanol und Wasser angereicherten entgasten Roh-Methanolstrom (VI) erhält,

(d) aus dem entgasten Roh-Methanolstrom (VI) aus Stufe (c) in einer Destillationsvorrichtung (D) einen Kohlendioxid und Dimethylether enthaltenden Leichtsiederstrom (VII) destillativ abtrennt und einen mit Methanol und Wasser angereicherten Sumpfstrom (VIII) erhält, und

(e) aus dem Sumpfstrom (VIII) aus Stufe (d) in einer weiteren Destillationsvorrichtung (E) einen Wasser enthaltenden Schwersiederstrom (IX) abtrennt und Methanol destillativ als Strom (X) gewinnt,
und welches dadurch gekennzeichnet ist, dass man

(f) die werthaltigen Komponenten Kohlenmonoxid, Kohlendioxid, Dimethylether und Methan der Ströme (IV) sowie von mindestens einem der beiden Ströme (V) und (VII) einer Verbrennungseinheit (F) zuführt und darin unter Zufuhr eines sauerstoffhaltigen Gases (XI), welches einen Sauerstoffgehalt von 30 bis 100 Vol.-% aufweist, verbrennt und kohlendioxidhaltiges Rauchgas (XII) bildet,

(g) aus dem kohlendioxidhaltigen Rauchgas (XII) aus Stufe (f) in einer Kohlendioxid-Rückgewinnungseinheit (G) unter Bildung eines Abgasstroms (XIII) einen mit Kohlendioxid angereicherten Strom (XIV) abtrennt, und

(h) den in der Kohlendioxid-Rückgewinnungseinheit (G) abgetrennten und mit Kohlendioxid angereicherten Strom (XIV) aus Stufe (g) zur Synthesegas-Erzeugungseinheit (A) der Stufe (a) und/oder zur Methanol-Syntheseeinheit (B) der Stufe (b) rückführt.

Das erfindungsgemäße Verfahren basiert auf der, dem Fachmann bekannten kontinuierlich betriebenen Methanolsynthese nach dem Niederdruckverfahren, bei der Synthesegas bei einem Druck von 5 bis 10 MPa abs in Gegenwart eines Methanol-Synthesekatalysators zu einem methanolhaltigen Reaktionsgemisch umgesetzt und nachfolgend stufenweise zur Isolierung des Methanols aufgearbeitet wird. Bei der stufenweisen Aufarbeitung werden dabei verschiedene Ströme abgetrennt, die noch werthaltigen Komponenten aus nicht umgesetzten Einsatzstoffen oder Nebenprodukten, wie beispielsweise Kohlenmonoxid, Kohlendioxid, Dimethylether, Methan oder weiteren Nebenprodukten enthalten. Kern der Erfindung ist die stoffliche Wiederverwendung des Kohlenstoffs dieser werthaltigen Komponenten zur weiteren Synthese von Methanol unter gleichzeitiger Vermeidung einer Kohlendioxid-Emission. Das erfindungsgemäße Verfahren ist im Folgenden näher erläutert.

Fig. 1 zeigt das Blockdiagramm einer allgemeinen Ausführungsform des erfindungsgemäßen Verfahrens, bei der alle drei Ströme (IV), (V) und (VII) zur Verbrennungseinheit (F) geführt werden. Darin bedeuten

(A) Synthesegas-Erzeugungseinheit
(B) Methanol-Syntheseeinheit
(C) Entspannungseinheit
(D) Destillationsvorrichtung
(E) Destillationsvorrichtung
(F) Verbrennungseinheit
(G) Kohlendioxid-Rückgewinnungseinheit

(I) kohlenstoffhaltiger Einsatzstoff
(II) Synthesegas
(III) Roh-Methanolstrom
(IV) Kohlenmonoxid, Kohlendioxid, Wasserstoff und Methan enthaltender gasförmiger Strom
(V) Kohlendioxid und Methan enthaltendes Entspannungsgas
(VI) Entgaster Roh-Methanolstrom
(VII) Leichtsiederstrom aus Destillationsvorrichtung (D)
(VIII) Sumpfstrom aus Destillationsvorrichtung (D)
(IX) Schwersiederstrom aus Destillationsvorrichtung (E)
(X) Methanol
(XI) Sauerstoffhaltiges Gas
(XII) Rauchgas

(XIII) Abgasstrom aus Kohlendioxid-Rückgewinnungseinheit (G)
(XIV) Mit Kohlendioxid-angereicherter Strom aus Kohlendioxid-Rückgewinnungseinheit (G)

Die gestrichelten Linien in Strom (XIV) versinnbildlichen, dass Strom (XIV) erfindungsgemäß sowohl zur Synthesegas-Erzeugungseinheit (A) der Stufe (a) also auch zur Methanol-Syntheseeinheit (B) der Stufe (b) oder auch aufgesplittet zu beiden Einheiten (A) und (B) rückgeführt werden kann.

Als Synthesegas erzeugende Einsatzstoffe können beim erfindungsgemäßen Verfahren die verschiedensten kohlenstoffhaltigen Einsatzstoffe eingesetzt werden, unabhängig davon, ob diese fest, flüssig oder gasförmig vorliegen, und unabhängig von deren chemischer Natur. So können zur Erzeugung von Synthesegas beispielsweise sowohl Kohle als auch Kohlenwasserstoffe sowie Kohlenstoff und Wasserstoff enthaltende Verbindungen eingesetzt werden. Als bevorzugte kohlenstoffhaltige Einsatzstoffe genannt seien Erdgas, Biogas, Kohle, Holz, Kunststoffe, Erdöl, Bionaphtha oder kohlenwasserstoffhaltige Ströme aus der Erdöl- oder Erdgasverarbeitung, aus chemischen Produktionsverfahren, aus nachwachsenden Rohstoffen oder aus dem Kunststoff-Recycling. Im Falle von Kohle oder Holz erfolgt die Synthesegas-Erzeugung beispielsweise durch einen Vergasungsprozess, auch Kohlevergasung beziehungsweise Holzvergasung genannt. Geeignete Einsatzstoffe aus der Erdöl- oder Erdgasverarbeitung sind beispielsweise Naphtha, LPG, Benzin, Schweröl oder Vakuumrückstand. Unter kohlenwasserstoffhaltige Ströme aus chemischen Produktionsverfahren sind beispielsweise kohlenwasserstoffhaltige Ströme zu verstehen, die als Nebenprodukte anfallen und anstelle einer rein thermischen Verwertung auch als Einsatzstoff zur Erzeugung von Synthesegas eingesetzt werden können.

Besonders bevorzugt ist der Einsatz von Methan-haltigen Strömen und ganz besonders bevorzugt von Erdgas oder Biogas. Dabei ist es möglich und sogar vorteilhaft, im Erdgas sowie vor allem im Biogas vorhandenes Kohlendioxid ebenfalls der Synthesegas-Erzeugungseinheit (A) zuzuführen.

Aus dem kohlenstoffhaltigen Einsatzstoff (I) wird beim erfindungsgemäßen Verfahren in der sogenannten Synthesegas-Erzeugungseinheit (A) zunächst ein Kohlenmonoxid, Kohlendioxid und Wasserstoff enthaltendes Synthesegas (II) erzeugt. Als Erdgas (I) können beispielsweise beim erfindungsgemäßen Verfahren grundsätzlich alle Erdgase eingesetzt werden, die für die Erzeugung von Synthesegas als Rohstoff zur Methanolsynthese geeignet sind. Erdgas enthält üblicherweise 75 bis 100 Vol.-% Methan. Als Begleitstoffe neben Methan seien vor allem die höheren Kohlenwasserstoffe Ethan, Propan, Butan aber auch Ethen genannt. Ebenso können beim erfindungsgemäßen Verfahren grundsätzlich alle Biogase eingesetzt werden, die für die Erzeugung von Synthesegas als Rohstoff zur Methanolsynthese geeignet sind. Biogas enthält üblicherweise 40 bis 75 Vol.-% Methan und als Begleitstoffe im Wesentlichen Kohlendioxid, Wasser, Stickstoff und Sauerstoff.

Üblicherweise erfolgt die Erzeugung des Synthesegases (II) durch die im technischen Maßstab üblicherweise verwendeten Erzeugungsverfahren, wobei hier natürlich auch die Art des kohlenstoffhaltigen Einsatzstoffes (I) eine Rolle spielt. Bei Methan-haltigen Einsatzstoffen, wie etwa im Falle von Erdgas oder Biogas, erzeugt man in Stufe (a) das Synthesegas (II) bevorzugt durch Dampfreformierung, durch autotherme Reformierung, durch eine Kombination aus Dampfreformierung und autothermer Reformierung oder durch partielle Oxidation .

Ein besonderer Vorteil der partiellen Oxidation liegt darin, dass der Synthesegas-Erzeugungseinheit kein separates Brenngas zugeführt werden muss und somit auch kein Kohlendioxid-haltiges Rauchgas gebildet wird. Bei der partiellen Oxidation wird die zur Synthesegas-Erzeugung erforderliche Energie direkt durch partielle Oxidation aus dem Methan-haltigen Einsatzstoff gewonnen und die dabei gebildeten Verbrennungsgase Kohlendioxid und Kohlenmonoxid ebenfalls in der Methanolsynthese verwendet. Daher ist die partielle Oxidation von Methan-haltigen Strömen, wie beispielsweise von Erdgas oder Biogas beim erfindungsgemäßen Verfahren bevorzugt.

Die üblicherweise zu verwendenden Verschaltungen, Apparate, Verfahrensparameter, Hilfsstoffe sowie die Aufarbeitung des erhaltenen Roh-Synthesegases sind dem Fachmann wohlbekannt und im Stand der Technik ausführlich beschrieben.

Das erzeugte Synthesegas (II) enthält Kohlenmonoxid, Kohlendioxid und Wasserstoff, wobei deren Konzentration zusammen üblicherweise 50 bis 100 Vol.-%, bevorzugt ≥ 80 Vol.-% und besonders bevorzugt ≥ 90 Vol.-% beträgt. Als mögliche Begleitstoffe seien vor allem nicht-umgesetzte Bestandteile des eingesetzten kohlenstoffhaltigen Einsatzstoffs sowie Nebenprodukte aus dessen Umsetzung genannt, wie beispielsweise Stickstoff, Argon, Wasser oder Methan. Üblicherweise enthält das Synthesegas (II) neben Kohlenmonoxid, Kohlendioxid und Wasserstoff herstellungsbedingt auch Methan, sowie Stickstoff und Argon, welches beispielsweise durch den Einsatz von Luft in der Synthesegaserzeugung eingebracht wurde.

Je nach der Art der Erzeugung des Synthesegases (II) enthält dieses unterschiedliche Mengen an Kohlenmonoxid, Kohlendioxid und Wasserstoff. Die für die Methanolsynthese relevante und übliche Kenngröße zur Spezifizierung des Synthesegases ist die Stöchiometriezahl S, welche eingangs bereits erwähnt wurde und definiert ist als

$$S = \frac{n(H_2) - n(CO_2)}{n(CO) + n(CO_2)} \qquad (3)$$

wobei n jeweils für die jeweiligen Molmengen steht. Die in Stufe (a) erzeugten und in Stufe (b) einzusetzenden Synthesegase (II) können hinsichtlich ihrer Stöchiometriezahl S in einem breiten Bereich variieren. Im Allgemeinen setzt man in der Methanol-Syntheseeinheit (B) jedoch ein Synthesegas (II) mit einer Stöchiometriezahl S im Bereich von 1 bis 5, bevorzugt $\geq 1{,}3$, besonders bevorzugt $\geq 1{,}5$ und ganz besonders bevorzugt $\geq 2$, sowie bevorzugt $\leq 4$, besonders bevorzugt $\leq 3$ und ganz besonders bevorzugt $\leq 2{,}5$ ein.

Die Umsetzung des Synthesegases (II) erfolgt in einer sogenannten Methanol-Syntheseeinheit (B) bei einer Temperatur von 150 bis 300°C und einem Druck von 5 bis 10 MPa abs in Gegenwart eines Methanol-Synthesekatalysators. Das Synthesegas (II) wird hierzu üblicherweise durch einen Kompressor auf den gewünschten Druck verdichtet und in einem Reaktor unter den genannten Bedingungen umgesetzt.

Die Umsetzung erfolgt bevorzugt bei einer Temperatur von $\geq 170$°C und besonders bevorzugt bei $\geq 190$°C sowie bevorzugt bei $\leq 280$°C und besonders bevorzugt bei$\leq 260$°C. Hinsichtlich des Drucks erfolgt die Umsetzung bevorzugt bei $\geq 6$ MPa abs sowie bevorzugt bei $\leq 9$ MPa abs.

Als Reaktoren können grundsätzlich alle Reaktoren eingesetzt werden, die für die exotherm verlaufende Umsetzung von Synthesegas zu Methanol unter den genannten Verfahrensbedingungen geeignet sind. Reaktoren zur Synthese von Methanol aus Synthesegas sind dem Fachmann allgemein bekannt. Beispielhaft seien hierzu die in Ullmann's Encyclopedia of Industrial Chemistry, Kapitel "Methanol", Sektion 5.2.1 "Reactor Design", 2012 Wiley-VCH Verlag GmbH & Co. KGaA, Weinheim, Germany genannten adiabaten und quasi-isothermen Reaktoren, variobar Reaktoren und sogenannte doppelwandige Superconverter erwähnt.

Als Methanol-Synthesekatalysatoren können beim erfindungsgemäßen Verfahren grundsätzlich alle Katalysatoren eingesetzt werden, die für Umsetzung von Synthesegas zu Methanol unter den genannten Verfahrensbedingungen geeignet sind. Entsprechende Methanol-Synthesekatalysatoren sind dem Fachmann allgemein bekannt. Beispielhaft seien hierzu die in Ullmann's Encyclopedia of Industrial Chemistry, Kapitel "Methanol", Sektion 4.2 "Catalysts", 2012 Wiley-VCH Verlag GmbH & Co. KGaA, Weinheim, Germany genannten Kupfer und Zink-haltigen Heterogenkatalysatoren erwähnt. Im Allgemeinen enthalten diese noch weitere Elemente, wie beispielsweise Aluminium, seltene Erden oder Chrom.

Bei der Umsetzung des Kohlenmonoxid, Kohlendioxid und Wasserstoff enthaltenden Synthesegases (II) bilden sich Methanol und Wasser entsprechend der Reaktionsgleichungen (1) und (2)

$$CO + 2\,H_2 \rightleftharpoons CH_3OH \quad (1)$$

$$CO_2 + 3\,H_2 \rightleftharpoons CH_3OH + H_2O \quad (2)$$

Als typisches Nebenprodukt bildet sich Dimethylether. Zudem entsteht durch vollständige Hydrierung von Kohlenmonoxid beziehungsweise Kohlendioxid auch Methan als weiteres Nebenprodukt. Das im Reaktor erzeugte Reaktionsgemisch enthält somit Methanol, Wasser, Dimethylether, Kohlenmonoxid, Kohlendioxid, Wasserstoff und Methan. Des Weiteren bilden sich unter den genannten Reaktionsbedingungen jedoch üblicherweise auch noch weitere Nebenprodukte, wie beispielsweise Methylformiat, Essigsäure, höhere Alkohole mit Kohlenstoffzahlen von $\geq 2$, Ester und Ether mit Kohlenstoffzahlen von $> 2$, sowie auch Paraffine.

Zur Auftrennung des komplexen Reaktionsgemischs wird zunächst ein mit Methanol und Wasser angereicherter Roh-Methanolstrom (III) auskondensiert. Hierzu wird üblicherweise das im Reaktor erzeugte Reaktionsgemisch einem Kondensator zugeführt. Als Kondensatoren können die dem Fachmann bekannten Apparate eingesetzt werden, welche geeignet sind, unter den vorliegenden Bedingungen durch eine gezielte Abkühlung ein mit Methanol und Wasser angereichertes Kondensat zu erhalten. Im Allgemeinen wird das Reaktionsgemisch auf eine Temperatur unterhalb des Taupunktes von Methanol abgekühlt. Entsprechend der Löslichkeiten und Dampfdrücke der im Reaktionsgemisch enthaltenen Komponenten enthält der mit Methanol und Wasser angereicherte Roh-Methanolstrom (III) noch darin gelöste Gase wie beispielsweise Wasserstoff, Kohlenmonoxid, Kohlendioxid, Dimethylether, Methan, sowie höher als Methanol siedende Komponenten. Der auskondensierte Roh-Methanolstrom (III) wird dann zur weiteren Aufarbeitung aus der Methanol-Syntheseeinheit (B) ausgeschleust und zu Stufe (c) geführt.

Der nicht auskondensierte Gasstrom enthält insbesondere die nicht umgesetzten Einsatzstoffe Kohlenmonoxid, Kohlendioxid und Wasserstoff, sowie Methan. Um einen hohen Partialdruck der Synthesegaskomponenten Wasserstoff, Kohlenmonoxid und Kohlendioxid zu erhalten, wird üblicherweise ein Teil des nicht auskondensierten Gasstroms ausgeschleust. Wenn es erforderlich ist, wird dieser ausgeschleuste Gasstrom einer Wasserstoffabtrennung zugeführt um den Partialdruck von Wasserstoff im Reaktor zu erhöhen. Ein höherer Partialdruck von Wasserstoff im Reaktor reduziert die Bildung von Nebenkomponenten und drängt insbesondere auch die Fischer-Tropsch-Reaktion zurück. Der überwiegende Teil des nicht auskondensierten Gasstroms wird in die Methanol-Syntheseeinheit als Kreislaufgas zurückgeführt und über den Methanol-Synthesekatalysator geleitet um eine möglichst gute Ausnutzung des Synthesegases und somit hohe Ausbeuten an Methanol zu erzielen.

Demgemäß enthält die Methanol-Syntheseeinheit (B) in Stufe (b) vorteilhafterweise einen Kompressor zur Verdichtung des Synthesegases (II), einen Reaktor zur Umsetzung des Synthesegases (II), einen Kondensator zur Auskondensation des Roh-Methanolstroms (III) und eine Leitung zur Rückführung von nicht auskondensiertem Gas zum Reaktor.

Abhängig von der Zusammensetzung des zugeführten Synthesegases (II) ist es somit bereits möglich, am Reaktoreingang eine Stöchiometriezahl S von > 2 einzustellen. Bevorzugt stellt man beim erfindungsgemäßen Verfahren durch rückgeführtes Synthesekreisgas und gegebenenfalls auch durch die zusätzliche Rückführung von Wasserstoff aus der weiteren Aufarbeitung des ausgeschleusten Synthesekreisgases sowie der Zuführung von Frisch-Wasserstoff zur Methanol-Syntheseeinheit (B) eine Stöchiometriezahl S am Reaktoreingang von bevorzugt ≥ 2,5 und besonders bevorzugt von ≥ 2,8 sowie bevorzugt ≤ 4 und besonders bevorzugt von ≤ 3,8 ein.

Der nicht als Synthesekreisgas rückgeführte, nicht auskondensierte Gasstrom wird als gasförmiger Strom (IV) aus der Methanol-Syntheseeinheit (B) ausgeschleust und zur erfindungsgemäßen Stufe (f) geführt. Die Menge an auszuschleusenden Gasstrom (IV) ergibt sich aus der Massenbilanz der, der Methanol-Syntheseeinheit (B) zugeführten und abgeführten Ströme.

In Stufe (c) des erfindungsgemäßen Verfahrens wird der in Stufe (b) auskondensierte und aus der Methanol-Syntheseeinheit (B) ausgeschleuste Roh-Methanolstrom (III) in einer Entspannungseinheit (C) auf einen Druck von 0,1 bis 2 MPa abs entspannt und ein Kohlendioxid und Methan enthaltendes Entspannungsgas (V) sowie ein mit Methanol und Wasser angereicherter entgaster Roh-Methanolstrom (VI) erhalten. Die Entspannung erfolgt üblicherweise in einen Apparat, in dem sich Gasphase und Flüssigphase gut voneinander trennen lassen. Üblicherweise handelt es sich bei dem Apparat um einen Flüssigkeitsabscheider. Geeignete Apparate hierfür sind dem Fachmann bekannt.

Die Entspannung erfolgt bevorzugt auf einen Druck von ≥ 0,2 MPa abs und besonders bevorzugt auf ≥ 0,4 MPa abs sowie bevorzugt auf ≤ 1,5 MPa abs und besonders bevorzugt auf ≤ 1 MPa abs. Im Allgemeinen beträgt die Temperatur des entspannten Gemisches 0 bis 150°C, bevorzugt ≥ 10°C und besonders bevorzugt ≥ 20°C sowie bevorzugt ≤ 120°C und besonders bevorzugt ≤ 60°C.

Der entgaste Roh-Methanolstrom (VI) ist an Methanol und Wasser weiter angereichert, enthält jedoch entsprechend der Löslichkeiten und Dampfdrücke der im Roh-Methanolstrom (III) enthaltenen Komponenten auch noch weitere Komponenten, wie beispielsweise darin gelöste Gase wie Wasserstoff, Kohlenmonoxid, Kohlendioxid, Dimethylether, Methan oder höher als Methanol siedende Komponenten.

Das Kohlendioxid und Methan enthaltende Entspannungsgas (V) wird bevorzugt zur erfindungsgemäßen Stufe (f) geführt. Alternativ kann das Entspannungsgas (V) jedoch auch aus der Methanol-Syntheseanlage ausgeleitet und beispielsweise thermisch verwertet oder anderweitig entsorgt werden. Bevorzugt ist jedoch dessen Nutzung innerhalb der Methanol-Syntheseanlage als Zufuhrstrom zur Verbrennungseinheit (F).

In Stufe (d) des erfindungsgemäßen Verfahrens wird der in Stufe (c) erhaltene entgaste Roh-Methanolstrom (VI) in einer Destillationsvorrichtung (D) in einen Kohlendioxid und Dimethylether enthaltenden Leichtsiederstrom (VII) und einen mit Methanol und Wasser angereicherten Sumpfstrom (VIII) destillativ getrennt. Als Destillationsvorrichtungen kommen grundsätzlich die dem Fachmann für derartige Trennaufgaben bekannten, beziehungsweise mit seinem allgemeinen Fachkönnen auszulegende Vorrichtungen in Frage. Üblicherweise erfolgt die destillative Trennung in Stufe (d) in einer einzigen Destillationskolonne, wobei es natürlich auch möglich ist, mehrere Destillationskolonnen parallel zu betreiben. Neben dem eigentlichen Kolonnenkörper mit Einbauten enthält die Destillationskolonne wie üblich noch einen Kopfkondensator und einen Sumpfverdampfer. Der Kolonnenkörper kann beispielsweise mit Packungen, Füllkörpern oder Böden bestückt sein. Die Destillationsvorrichtung (D) kann mit dem allgemeinen Wissen des Fachmanns ausgelegt und betrieben werden.

Der destillativ abgetrennte Leichtsiederstrom (VII) enthält vornehmlich Kohlendioxid und Dimethylether als abgetrennte Leichtsieder und basierend auf der Zusammensetzung des entgasten Roh-Methanolstroms (VI) noch weitere Leichtsieder, wie beispielsweise Methan, sowie abhängig von Trennleistung und Fahrweise der Destillationsvorrichtung auch Methanol oder höher als Methanol siedende Komponenten wie beispielsweise Wasser. Der Kohlendioxid und Dimethylether enthaltende Leichtsiederstrom (VII) wird ebenfalls bevorzugt zur erfindungsgemäßen Stufe (f) geführt. Falls das Entspannungsgas (V) zur Verbrennungseinheit (F) geleitet und somit bereits innerhalb der Methanol-Syntheseanlage wiederverwendet wird, kann der Leichtsiederstrom (VII) alternativ auch aus der Methanol-Syntheseanlage ausgeleitet und beispielsweise thermisch verwertet oder anderweitig entsorgt werden. Bevorzugt ist jedoch dessen Nutzung innerhalb der Methanol-Syntheseanlage als Zufuhrstrom zur Verbrennungseinheit (F).

Der mit Methanol und Wasser angereicherten Sumpfstrom (VIII) enthält zudem auch noch weitere, höher als Methanol siedende Komponenten, wie beispielsweise höher als Methanol siedende Nebenprodukte aus der Methanolsynthese, wie etwa Essigsäure, höhere Alkohole, höhere Ester, höhere Ether oder Paraffine.

Zur Gewinnung des Methanols wird schließlich in Stufe (e) von dem in Stufe (d) erhaltenen Sumpfstrom (VIII)

in einer weiteren Destillationsvorrichtung (E) ein Wasser enthaltender Schwersiederstrom (IX) abgetrennt und Methanol destillativ als Strom (X) gewonnen. Als Destillationsvorrichtungen kommen für Stufe (e) grundsätzlich die dem Fachmann für derartige Trennaufgaben bekannten, beziehungsweise mit seinem allgemeinen Fachkönnen auszulegende Vorrichtungen in Frage. Grundsätzlich kann die destillative Trennung in Stufe (e) in einer einzigen Destillationskolonne erfolgen. Neben dem eigentlichen Kolonnenkörper mit Einbauten enthält die Destillationskolonne wie üblich noch einen Kopfkondensator und einen Sumpfverdampfer. Der Kolonnenkörper kann beispielsweise mit Packungen, Füllkörpern oder Böden bestückt sein. Es ist natürlich auch möglich, mehrere Destillationskolonnen parallel zu betreiben oder das Methanol in mehreren Destillationskolonnen stufenweise abzutrennen. Die Destillationsvorrichtung (E) kann mit dem allgemeinen Wissen des Fachmanns ausgelegt und betrieben werden.

Methanol wird als Strom (X) üblicherweise als Kopfprodukt gewonnen. Es ist aber grundsätzlich auch möglich, Methanol als sogenannten Seitenstrom zu gewinnen und als Kopfstrom noch vorhandene Leichtsieder abzutrennen.

Eine energetisch attraktive Variante ist die sogenannte Zweidruckdestillation. Bei dieser sind zwei Destillationskolonnen hintereinander geschaltet und energetisch miteinander gekoppelt. Die erste Destillationskolonne wird dabei unter Druck, üblicherweise bei 0,5 bis 1,5 MPa abs betrieben und Methanol als Leichtsieder über Kopf abgetrennt. Die erste Destillationskolonne wird dabei so betrieben, dass ein Teil des Methanols im Sumpf verbleibt und dieser einer zweiten Destillationskolonne zugeführt wird. Die zweite Destillationskolonne wird bei einem niedrigeren Druck, beispielsweise Atmosphärendruck, betrieben. Der Sumpf der zweiten Destillationskolonne ist dabei energetisch mit dem Leichtsiederstrom der ersten Destillationskolonne gekoppelt, das heißt, die bei der Abkühlung des Leichtsiederstroms der ersten Destillationskolonne abgegebene Wärmeenergie dient zur Aufheizung der zweiten Destillationskolonne. Auch in der zweiten Destillationskolonne wird Methanol als Leichtsieder über Kopf abgetrennt. Die im Sumpf der zweiten Destillationskolonne anfallenden Schwersieder werden abgetrennt und ausgeschleust. Aufbau und Betrieb der Zweidruckdestillation und insbesondere der Zweidruckdestillation zur Gewinnung von Methanol sind dem Fachmann bekannt.

Der Schwersiederstrom (IX) enthält Wasser sowie auch noch weitere, höher als Methanol siedende Komponenten, wie beispielsweise höher als Methanol siedende Nebenprodukte aus der Methanolsynthese, wie etwa Essigsäure, höhere Alkohole, höhere Ester, höhere Ether oder Paraffine. Dieser Strom kann beispielsweise einer Abwasserbehandlung zugeführt werden.

Über Strom (X) kann Methanol in einer hohen Reinheit von ≥ 95 Gew.-%, bevorzugt ≥ 98 Gew.-% und besonders bevorzugt ≥ 99 Gew.-% gewonnen werden. Als Begleitstoffe seien Restmengen an destillativ nicht vollständig abgetrennten Leicht- und Schwersiedern zu nennen, insbesondere Wasser, und sehr geringe Mengen an Ethanol, Ester und Ether.

Bei der stufenweisen Aufarbeitung des Reaktionsgemischs zur Gewinnung des Methanols als Strom (X) werden die Ströme (IV), (V) und (VII) abgetrennt. Diese enthalten jedoch noch werthaltige Komponenten, wie beispielsweise Kohlenmonoxid, Kohlendioxid, Methan und Dimethylether. Kern der Erfindung ist die weitgehende stoffliche Wiederverwendung des Kohlenstoffs dieser werthaltigen Komponenten zur weiteren Synthese von Methanol unter gleichzeitiger Vermeidung einer Kohlendioxid-Emission.

Die erfindungswesentlichen Stufen (f) bis (h) sind im Folgenden näher erläutert.

Beim erfindungsgemäßen Verfahren werden

(f) die werthaltigen Komponenten Kohlenmonoxid, Kohlendioxid, Dimethylether und Methan der Ströme (IV) sowie von mindestens einem der beiden Ströme (V) und (VII) einer Verbrennungseinheit (F) zugeführt und darin unter Zufuhr eines sauerstoffhaltigen Gases (XI), welches einen Sauerstoffgehalt von 30 bis 100 Vol.-% aufweist, verbrannt und ein kohlendioxid-haltiges Rauchgas (XII) gebildet,

(g) aus dem kohlendioxidhaltigen Rauchgas (XII) aus Stufe (f) in einer Kohlendioxid-Rückgewinnungseinheit (G) unter Bildung eines Abgasstroms (XIII) ein mit Kohlendioxid angereicherter Strom (XIV) abgetrennt, und

(h) der in der Kohlendioxid-Rückgewinnungseinheit (G) abgetrennte und mit Kohlendioxid angereicherte Strom (XIV) aus Stufe (g) zur Synthesegas-Erzeugungseinheit (A) der Stufe (a) und/oder zur Methanol-Syntheseeinheit (B) der Stufe (b) zurückgeführt.

Bevorzugt werden in Stufe (f) die werthaltigen Komponenten Kohlenmonoxid, Kohlendioxid, Dimethylether und Methan der Ströme (IV), (V) und (VII) der Verbrennungseinheit (F) zugeführt.

Da die werthaltigen Komponenten Methan und Dimethylether der Ströme (IV), (V) und (VII), aber auch andere darin enthaltene kohlenstoffhaltige Nebenprodukte nicht direkt in dieser Form als Edukte für die Methanolsynthese eingesetzt werden können, ist es erforderlich, diese zuerst chemisch in eine geeignete Form umzuwandeln. Die erfindungsgemäße Lösung sieht daher vor, diese in einer Verbrennungseinheit (F) unter Bildung eines kohlendioxidhaltigen Rauchgases zu verbrennen. Durch die Umwandlung in Kohlendioxid ist es möglich, auch die werthaltigen Komponenten Methan, Dimethylether und weitere kohlenstoffhaltige Nebenprodukte in der Methanol-synthese als Edukt erneut einzusetzen. Daher werden beim erfindungsgemäßen Verfahren die Ströme (IV), (V) und (VII) zunächst einer Verbrennungseinheit (F) zugeführt. In dieser werden die brennbaren werthaltigen Komponenten unter Zufuhr eines sauerstoffhaltiges Gas (XI), welches einem Sauerstoffgehalt von 30 bis 100 Vol.-% aufweist, verbrannt. Die Verbrennung erfolgt dabei üblicherweise in einer sogenannten Brennkammer unter Bildung von Kohlendioxid und Wasser. Üblicherweise erfolgt die Verbrennung bei Atmosphärendruck. Es ist aber auch möglich, die Verbrennung bei einem niedrigeren oder höheren Druck durchzuführen. Der Vollständigkeit halber sei hierfür ein Druckbereich von 0,05 bis 0,5 MPa abs genannt.

Als Brennkammern können grundsätzlich alle Apparate eingesetzt werden, die für die stark exotherm verlaufende Oxidation eines entsprechenden Kohlenmonoxid, Methan und Dimethylether enthaltenden Stroms mit einem sauerstoffhaltigen Gas mit einem entsprechenden Sauerstoffgehalt geeignet sind. Geeignete Brennkammern sind dem Fachmann bekannt beziehungsweise können mit dem allgemeinen Wissen des Fachmanns ausgelegt und betrieben werden. Beispielhaft genannt seien etwa adiabate Brennkammern oder Reaktoren mit Wärmeauskoppelungen wie direkt befeuerte Dampferzeuger, etwa in Form von Wasserrohrkesseln oder Flammrohr- beziehungsweise Rauchrohrkesseln.

Ein wesentlicher Vorteil des erfindungsgemäßen Einsatzes eines sauerstoffhaltigen Gases (XI) mit einem Sauerstoffgehalt von 30 bis 100 Vol.-% ist ein deutlich höherer Gehalt an Kohlendioxid im erhaltenen Verbrennungsgas im Vergleich zum Einsatz von Luft mit nur etwa 21 Vol.-% Sauerstoff. Entsprechend ist beim erfindungsgemäßen Verfahren gegenüber Luft als Oxidator natürlich auch der Anteil unerwünschter Inertgase, wie etwa Stickstoff oder Argon, deutlich geringer.

Bevorzugt führt man der Verbrennungseinheit (F) ein sauerstoffhaltiges Gases (XI) mit einem Sauerstoffgehalt von $\geq$ 50 Vol.-%, besonders bevorzugt von $\geq$ 80 Vol.-%, ganz besonders bevorzugt von $\geq$ 90 Vol.-%, vor allem von $\geq$ 95 Vol.-% und insbesondere reinen Sauerstoff zu. Beim Einsatz von reinem Sauerstoff in der Verbrennung spricht man auch von einem sogenannten Oxyfuel-Verfahren.

Üblicherweise werden somit $\geq$ 90%, bevorzugt $\geq$ 95%, besonders bevorzugt $\geq$ 98% und ganz besonders bevorzugt $\geq$ 99% des Kohlenstoffs der brennbaren Komponenten in Kohlendioxid umgewandelt.

Ein weiterer Vorteil durch den Einsatz eines Sauerstoff-reichen Gases als Oxidator anstelle von Luft ist auch die deutliche Reduzierung von thermisch gebildeten Stickoxiden $NO_x$. Im Fall des Einsatzes von reinem Sauerstoff und eines nur sehr geringen Gehalts an Stickstoff über die zugeführten Ströme (IV), (V) und (VII) kann die Bildung von Stickoxiden $NO_x$ sogar nahezu komplett vermieden werden.

Des Weiteren sind Verbrennungsprozesse mit Sauerstoff-reichen Gasen als Oxidator im Vergleich zur Luft aufgrund der höheren Verbrennungstemperaturen energetisch effizienter. So ergibt sich beispielsweise bei der Verbrennung von reinem Methan beim Einsatz von Luft eine rechnerische Verbrennungstemperatur von ca. 1800 bis 2000°C, wohingegen die Verbrennung von reinem Methan mit reinem Sauerstoff eine rechnerische Verbrennungstemperatur von ca. 4000 bis 5000°C ergibt. Um, vor allem auch aus materialtechnischen Gründen, einer besonders hohen Verbrennungstemperatur gegenzusteuern, wird bei der erfindungsgemäßen Verbrennung üblicherweise ein Teil des Rauchgases zur Temperatursteuerung, nach Abkühlen, in die Brennkammer rückgeführt.

Da beim Einsatz eines sauerstoffhaltigen Gases (XI) mit entsprechend hohem Sauerstoffgehalt das gebildete Rauchgas nur einen entsprechend geringen Gehalt an Stickstoff aufweist, beziehungsweise sogar praktisch frei von Stickstoff ist, reicht im allgemeinen auch eine entsprechend kleine Trennstufe zur Abtrennung des Stickstoffs vom Kohlendioxid aus.

Um den Gehalt an Kohlendioxid im erhaltenen Verbrennungsgas weiter zu erhöhen, ist es in Allgemeinen vorteilhaft, vor der Ausschleusung aus der Verbrennungseinheit (F) den Gehalt an Wasser zu verringern. Üblicherweise erfolgt dies durch einfache Auskondensation in einem Kondensator. Als Kondensatoren können die dem Fachmann bekannten Apparate eingesetzt werden, welche geeignet sind, unter den vorliegenden Bedingungen durch eine gezielte Abkühlung Wasser als Kondensat von einem Kohlendioxid und wasserhaltigen Gasstrom abzutrennen. Im Allgemeinen wird das Verbrennungsgas auf eine Temperatur unterhalb des Taupunktes von Wasser abgekühlt. Wird Wasser auskondensiert, so wird dieses als Strom (XV), und das verbleibende, von Wasser abgereicherte Verbrennungsgas als kohlendioxidhaltiges Rauchgas (XII) aus der Verbrennungseinheit (F) ausgeschleust. Bevorzugt werden 50 bis 100%, besonders bevorzugt $\geq$ 80% und ganz besonders bevorzugt $\geq$ 90% des im Verbrennungsgas vorhandenen Wassers auskondensiert.

Bevorzugt ist somit ein Verfahren, bei dem die Verbrennungseinheit (F) in Stufe (f) eine Brennkammer und einen Kondensator enthält, man aus dem in der Brennkammer erhaltenen Verbrennungsgas im Kondensator Wasser auskondensiert und als Strom (XV) aus der Verbrennungseinheit (F) abführt, und der verbleibende gasförmige Strom das kohlendioxidhaltige Rauchgases (XII) bildet. Fig. 2 zeigt ein Blockdiagramm einer allgemeinen Ausführungsform, bei der Wasser aus dem Verbrennungsgas auskondensiert und als Strom (XV) abgeführt wird.

Es ist jedoch auch möglich, wenngleich auch nicht bevorzugt, das Wasser vollständig im Verbrennungsgas zu belassen und dieses zusammen mit den Kohlendioxid als kohlendioxidhaltiges Rauchgas (XII) aus der Verbrennungseinheit (F) auszuschleusen.

Eine Abschätzung verdeutlicht die signifikante Erhöhung des Kohlendioxidgehalts im Rauchgas (XII) durch den Einsatz von reinem Sauerstoff gegenüber Luft als Oxidator, sowie durch die nachfolgende Abtrennung des Wassers.

So wird bei einer theoretischen Abschätzung der Verbrennung von reinem Methan zu Kohlendioxid und Wasser im Falle von Luft als Oxidator ein Kohlendioxidgehalt im Verbrennungsgas von rund 9 Vol.-%, und selbst nach vollständiger Abtrennung des Wassers von nur 12 Vol.-% erreicht. Dagegen führt der Einsatz von reinem Sauerstoff als Oxidator bereits zu einem Kohlendioxidgehalt im Verbrennungsgas von rund 33 Vol.-%. Durch vollständige Auskondensation des Wassers kann sogar ein rechnerischer Kohlendioxidgehalt von 100 Vol.-% erreicht werden. Dies entspricht einer Erhöhung des Kohlendioxidgehalts beim Einsatz von reinem Sauerstoff gegenüber Luft ohne Abtrennung des Wassers um einen Faktor 3,7 (9 Vol.-% vs. 33 Vol.-%) sowie mit Abtrennung des Wassers sogar um einen Faktor 8,3 (12 Vol.-% vs. 100 Vol.-%). Abhängig von der tatsächlichen Zusammensetzung des, der Verbrennungseinheit (F) zugeführten Gases ergeben sich natürlich etwas abweichende Faktoren. Dennoch führt der Einsatz von reinem Sauerstoff als Oxidator jeweils zu einem signifikant höheren Kohlendioxidgehalt im Verbrennungsgas und insbesondere nach der Auskondensation von Wasser auch im ausgeschleusten Rauchgas (XII).

Insbesondere durch den Einsatz von reinem Sauerstoff als Oxidator weist das Rauchgas (XII) einen deutlich höheren Gehalt an der werthaltigen Komponente Kohlendioxid auf. Anders formuliert, wird durch den Einsatz von reinem Sauerstoff bei gleicher absoluter Kohlendioxidmenge wesentlich weniger Rauchgas gebildet. Somit kann beim erfindungsgemäßen Verfahren auch die Größe der Verbrennungseinheit (F) und insbesondere der Brennkammer und des Kondensators sowie nachfolgender Apparate und Leitungen entsprechend kleiner dimensioniert werden.

Neben Kohlendioxid als werthaltige Komponente enthält das erhaltene Rauchgas (XII) in der Regel noch diverse Inertgase, welche der Verbrennungseinheit (F) über die Ströme (IV), (V) und (VII), und/oder das sauerstoffhaltige Gas (XI) zugeführt wurden, wie beispielsweise Stickstoff oder Argon. Da auch hinsichtlich des zugeführten Sauerstoffs die Verbrennung üblicherweise nicht vollständig erfolgt, enthält das Rauchgas (XII) in der Regel auch nicht-umgesetzten Sauerstoff, üblicherweise im Bereich von 2 bis 20 Vol.-%. Je nachdem, ob und inwieweit Wasser aus dem Verbrennungsgas auskondensiert wurde, enthält das Rauchgas (XII) auch noch das bei der Verbrennung gebildete Wasser beziehungsweise den nach Auskondensation des Wassers nichtauskondensierten Anteil. Im Allgemeinen weist das aus der Verbrennungseinheit (F) ausgeschleuste Rauchgas (XII) einen Kohlendioxidgehalt von 25 bis 90 Vol.-%, bevorzugt $\geq$ 50 Vol.-%, besonders bevorzugt $\geq$ 60 Vol.-% und ganz besonders bevorzugt $\geq$ 70 Vol.-%, sowie bevorzugt $\leq$ 85 Vol.-% und besonders bevorzugt $\leq$ 80 Vol.-% auf.

Aus dem in Stufe (f) gebildeten kohlendioxidhaltigen Rauchgas (XII) wird nun in Stufe (g) in einer Kohlendioxid-Rückgewinnungseinheit (G) unter Bildung eines Abgasstroms (XIII) ein mit Kohlendioxid angereicherter Strom (XIV) abgetrennt. Als Kohlendioxid-Rückgewinnungseinheit (G) können grundsätzlich alle Vorrichtungen und Verfahren eingesetzt werden, welche für die Abtrennung und Aufkonzentrierung von Kohlendioxid aus kohlendioxidhaltigen Gasströmen, welche neben Kohlendioxid noch Inertgase, wie etwa Stickstoff und Argon, sowie gegebenenfalls Wasser als weitere Komponenten enthalten, geeignet sind. Entsprechende Vorrichtungen und Verfahren sind dem Fachmann bekannt. Eine allgemeine Übersicht möglicher Vorrichtungen und Verfahren findet sich beispielsweise in Ullmann's Encyclopedia of Industrial Chemistry, Kapitel "Carbon Dioxide", Sektion 13.3 "CCS-related Separation Technologies", 2014 Wiley-VCH Verlag GmbH & Co. KGaA, Weinheim, Germany. Beispielhaft genannt seien die sogenannten Gaswäscheverfahren, bei denen das Kohlendioxid in einem sogenannten Gaswäsche-Lösungsmittel physikalisch oder chemisch absorbiert und nachfolgend wieder desorbiert wird, sowie Membranverfahren, bei denen das Kohlendioxid über eine Kohlendioxid-sensitive Membran abgetrennt wird.

Bei der physikalischen Absorption wird das Kohlendioxid in einem Absorber unter erhöhtem Druck in einem geeigneten Lösungsmittel wie etwa Methanol, N-Methylpyrrolidon oder Polyethylenglykoldimethylether absorbiert und nachfolgend in einem Desorber unter Entspannung wieder desorbiert. Das an Kohlendioxid abgereicherte Lösungsmittel wird üblicherweise wieder zum Absorber zurückgeführt. Typischerweise erfordert die physikalische Absorption Drücke im Bereich von etwa 2 bis 5 MPa abs. Da das kohlendioxidhaltige Rauchgas (XII) üblicherweise jedoch nur einen Druck von 0,1 bis 0,3 MPa abs aufweist, müsste dieses unter Aufwendung von Energie erst komprimiert werden.

Bevorzugt beim erfindungsgemäßen Verfahren ist daher die chemische Absorption in einem Lösungsmittel, welches Kohlendioxid chemisch bindet. Für die chemische Absorption sind höhere Drücke in der Regel nicht erforderlich, so dass das kohlendioxidhaltige Rauchgas (XII) nicht erst energieintensiv komprimiert werden muss. Als chemisch wirkende Lösungsmittel sind vor allem basische Lösungsmittel genannt. Bei der chemische Absorption wird in der Kohlendioxid-Rückgewinnungseinheit (G) in Stufe (g) Kohlendioxid aus dem kohlendioxidhaltigen Rauchgas (XII) in einem Absorber in einem basischen Lösungsmittel unter Bildung des Abgasstroms (XIII) absorbiert und nachfolgend in einem Desorber bei höherer Temperatur wieder desorbiert. Das an Kohlendioxid abgereicherte Lösungsmittel wird üblicherweise wieder zum Absorber zurückgeführt.

Bevorzugt ist daher ein Verfahren, bei dem man in der Kohlendioxid-Rückgewinnungseinheit (G) in Stufe (g) Kohlendioxid aus dem kohlendioxidhaltigen Rauchgas (XII) in einem Absorber in einem basischen Lösungsmittel unter Bildung des Abgasstroms (XIII) absorbiert, aus dem mit Kohlendioxid beladenen Lösungsmittel in einem Desorber einen mit Kohlendioxid angereicherten Strom (XIV) freigesetzt und das vom Kohlendioxid abgereicherte Lösungsmittel wieder zum Absorber zurückführt.

Bei den basischen Lösungsmitteln handelt es sich üblicherweise um wässrige Lösungen basischer anorganischer oder organischer Substanzen. Als basische anorganische Substanzen seien etwa Salze des Hydrogenkarbonats, und als basische organische Substanzen organische Amine genannt. Üblicherweise liegt der Wassergehalt der wässrigen Lösungen bei 20 bis 80 Gew.-%, bevorzugt bei ≥ 40 Gew.-% und besonders bevorzugt bei ≥ 50 Gew.-%.

Besonders bevorzugt setzt man beim erfindungsgemäßen Verfahren als basisches Lösungsmittel eine wässrige Lösung eines organischen Amins ein. Bei den Aminen kann es sich um lineare oder cyclische, unverzweigte oder verzweigte Verbindungen handeln. Geeignete Amine sind beispielsweise in US 2008/236,390, US 2010/192,770 oder US 2011/094,381 genannt. Bevorzugt weisen die organischen Amine eine Molmasse von 50 bis 500 g/mol auf. Besonders bevorzugt ist der Einsatz von Monoethanolamin, Piperazin, 2-Amino-2-methyl-1-propanol, Triethylendiamin, N-Methyldiethanolamin, tert.-Butylaminoethoxyethanol, und ganz besonders bevorzugt von N-Methyldiethanolamin.

Üblicherweise wird bei der chemischen Absorption das kohlendioxidhaltige Rauchgas (XII) im Absorber bei einer Temperatur von 10 bis 100°C, bevorzugt ≥ 20°C sowie bevorzugt ≤ 60°C und besonders bevorzugt ≤ 40°C mit dem basischen Lösungsmittel in Kontakt gebracht. Als Absorber können grundsätzlich alle Apparate eingesetzt werden, welche zur Absorption von Kohlendioxid aus einem kohlendioxidhaltigen Rauchgasstrom geeignet sind. Geeignete Apparate hierfür sind dem Fachmann bekannt und können mit dem üblichen Fachwissen ausgelegt werden. Üblicherweise werden zur Absorption sogenannte Absorptionskolonnen eingesetzt. Zum besseren Wärme- und Stoffaustausch enthalten diese vorteilhafterweise Packungen oder Böden. Bei Kontakt mit dem basischen Lösungsmittel wir das Kohlendioxid chemisch darin gebunden. Die nicht im basischen Lösungsmittel absorbierten Komponenten werden als Abgasstrom (XIII) als sogenannter Purge-Strom aus der Absorptionskolonne abgeführt. Das mit Kohlendioxid beladene Lösungsmittel wird am Absorbersumpf abgezogen, dem Desorber zugeführt und darin mit Wasserdampf gestrippt um das Kohlendioxid wieder freizusetzen. Vorteilhafterweise wird der Desorber bevorzugt bei einer um 40 bis 110°C höheren Temperatur betrieben als der Absorber. Geeignete Apparate hierfür sind dem Fachmann ebenfalls bekannt und können mit dem üblichen Fachwissen ausgelegt werden. Üblicherweise werden zur Desorption sogenannte Desorptionskolonnen eingesetzt. Zum besseren Wärme- und Stoffaustausch enthalten auch diese vorteilhafterweise Packungen oder Böden. Aus dem am Desorberkopf erhaltenen Desorptionsstrom wird vorteilhafterweise zunächst Wasser auskondensiert. Das auskondensierte Wasser kann dann beispielsweise wieder in den Kohlendioxid-Rückgewinnungsprozess rückgeführt werden. Das von Kohlendioxid und Wasser abgereicherte Lösungsmittel wird dann zum Absorber zurückgeführt.

Sowohl Absorber als auch Desorber werden üblicherweise bei 0,1 bis 0,3 MPa abs betrieben, die exakten Drücke richten sich in der Regel an den nachfolgenden Prozessschritten aus und können vom Fachmann leicht bestimmt werden. Alternativ kann die Absorption auch bei höheren Drucken betrieben werden. Die Kohlendioxid-Aufnahmekapazität des Lösungsmittels ist dann höher als bei Bedingungen nahe Umgebungsdruck. In der Folge ist die Regeneration im Desorber auch energieeffizienter. Allerdings müsste bei diese Variante das kohlendioxidhaltige Rauchgas (XII) verdichtet werden, was sich negativ auf die gesamte Energiebilanz auswirkt. Daher werden Absorber und Desorber bevorzugt bei 0,1 bis 0,3 MPa abs betrieben.

Da die Desorption üblicherweise bei höherer Temperatur erfolgt als die Absorption, ist das aus dem Absorber entnommene, mit Kohlendioxid beladene Lösungsmittel zu erwärmen und das aus dem Desorber entnommene, von Kohlendioxid und Wasser abgereicherte Lösungsmittel für dessen erneuten Einsatz im Absorber abzukühlen. Besonders vorteilhaft für die Energieeffizienz der Kohlendioxid-Rückgewinnung ist daher der Einsatz eines sogenannten Kreuzwärmetauschers, in dem das wärmere Lösungsmittel aus dem Desorber das kältere Lösungsmittel aus dem Absorber aufheizt. Durch den Einsatz weiterer Lösungsmittelwärmetauscher, lässt sich die Energieeffizienz des Verfahrens weiter steigern. Die genaue Einstellung der Temperatur des von Kohlendioxid abgereicherten Lösungsmittels für dessen Einsatz im Absorber kann dann beispielsweise durch einen Luft- oder Wasserkühler erfolgen.

Des Weiteren ist es möglich, die Energieeffizienz der Kohlendioxid-Rückgewinnung durch weitere Wärmeintegrationsmaßnahmen, wie beispielsweise durch den Einsatz eines Flüssigkeitskühler im unteren Absorberbereich, weiter zu steigern. Auch Lösungsmittelverluste können durch weitere Maßnahmen reduziert werden. So ist es beispielsweise möglich und bei Lösungsmitteln mit einem nicht zu vernachlässigenden Dampfdruck vorteilhaft, den Gasstrom im oberen Absorberbereich über ein Waschbett zu leiten und die flüssige Phase über eine Direktkühlung mit Umlaufkühler zu kühlen.

Um Verluste an Lösungsmittel auszugleichen, die sich trotz diverser Gegenmaßnahmen in der Regel nicht gänzlich vermeiden lassen, wird üblicherweise Lösungsmittel oder einzelne Lösungsmittelkomponenten als sogenannter Make-up Strom zugeführt. Somit ist es möglich, die Stände in den beiden Kolonnen zeitlich konstant zu halten. Üblicherweise erfolgt die Zufuhr am Absorberkopf.

Durch die Kohlendioxid-Rückgewinnungseinheit (G) wird das Kohlendioxid aus dem Rauchgas (XII) selektiv entfernt und in Form eines mit Kohlendioxid angereicherten Stroms (XIV) zur weiteren Verwendung in der Methanolsynthese rückgewonnen. Üblicherweise enthält der mit Kohlendioxid angereicherte Strom (XIV) Kohlendioxid in Höhe von 80 bis 100 Vol.-% auf wasserfreier Basis, bevorzugt ≥ 97 Vol.-% auf wasserfreier Basis, sowie geringe Mengen an Fremdgasen, wie beispielsweise Stickstoff, Sauerstoff oder Argon, welche jedoch üblicherweise unterhalb von 0,3 Vol.-% auf wasserfreier Basis liegen. Zudem enthält der mit Kohlendioxid angereicherte Strom (XIII) üblicherweise auch

Wasser, welches bei den oben genannten Vol.-% Angaben jedoch bereits herausgerechnet wurde.

Die genannten Kohlendioxid-Rückgewinnungsverfahren zeichnen sich durch eine relativ hohe Kohlendioxid-Rückgewinnungsrate aus, wobei unter der Kohlendioxid-Rückgewinnungsrate das Verhältnis zwischen der Menge des Kohlendioxids, welches der Kohlendioxid-Rückgewinnungseinheit (G) über das kohlendioxidhaltige Rauchgas (XII) zugeführt und der Menge des Kohlendioxids, welches aus der Kohlendioxid-Rückgewinnungseinheit (G) über den mit Kohlendioxid angereicherten Strom (XIV) abgeführt wird, zu verstehen ist. Durch die genannten Verfahren lassen sich Kohlendioxid-Rückgewinnungsraten von 90 bis fast 100% leicht erreichen. Bevorzugt liegt die Kohlendioxid-Rückgewinnungsrate bei ≥ 95%, besonders bevorzugt bei ≥ 98% und ganz besonders bevorzugt bei ≥ 99% sowie bevorzugt bei ≤ 99,9%.

Der Abgasstrom (XIII) enthält die nicht über den mit Kohlendioxid angereicherten Strom (XIV) abgetrennten Komponenten des Rauchgases (XII), insbesondere Inertgase, wie etwa Stickstoff oder Argon, aber auch Sauerstoff sowie nicht in der Kohlendioxid-Rückgewinnungseinheit (G) abgetrenntes Wasser. Über den Abgasstrom (XIII) werden somit vor allem gezielt Inertgase aus dem Methanol-Syntheseverfahren ausgetragen und somit einer unerwünschten Aufpegelung entgegengewirkt. Der Abgasstrom (XIII) fungiert somit nicht nur als Abgasstrom für die Kohlendioxid-Rückgewinnungseinheit (G) sondern übernimmt zugleich auch die wichtige Funktion des sogenannten Purge-Stroms zur Ausschleusung von Inertgasen aus der Methanolsynthese.

Der in der Kohlendioxid-Rückgewinnungseinheit (G) abgetrennte und mit Kohlendioxid angereicherten Strom (XIV) wird anschließend zur Synthesegas-Erzeugungseinheit (A) der Stufe (a) und/oder zur Methanol-Syntheseeinheit (B) der Stufe (b) rückgeführt. Die erfindungsgemäße Rückführung kann also sowohl zur Synthesegas-Erzeugungseinheit (A) der Stufe (a) also auch zur Methanol-Syntheseeinheit (B) der Stufe (b) oder auch aufgesplittet zu beiden erfolgen.

Im Falle der Rückführung des Kohlendioxids zur Synthesegas-Erzeugungseinheit (A) kann dieses beispielsweise dem kohlenstoffhaltigen Einsatzstoff (I) zugeführt und zusammen mit diesem dann zur Erzeugung des Synthesegases (II) eingesetzt werden. Je nach der Art der Synthesegas-Erzeugung durchläuft das Kohlendioxid die Synthesegas-Erzeugungseinheit (A) unverändert als Kohlendioxid oder wird mehr oder weniger durch Reaktion mit dem gebildeten Wasserstoff oder durch Reaktion mit zugefügtem oder gebildetem Wasser zu Kohlenmonoxid umgewandelt. Im Falle der Rückführung des Kohlendioxids zur Methanol-Syntheseeinheit (B) kann dieses beispielsweise dem Synthesegases (II) zugeführt und zusammen mit diesem dann zur Synthese von Methanol eingesetzt werden. Der Vollständigkeit halber sei genannt, dass im Falle einer Aufsplittung der Rückführung des Kohlendioxids das Splitverhältnis natürlich im gesamten Bereich zwischen 0 und 100% variieren kann. In allen genannten Fällen kann jedoch der Kohlenstoff des Kohlendioxids jeweils zur weiteren Synthese von Methanol stofflich verwertet werden.

Auch wenn Strom (XIV) problemlos zur Synthesegas-Erzeugungseinheit (A) der Stufe (a) rückgeführt werden kann, so ist es der Einfachheit halber vorteilhaft und bevorzugt, Strom (XIV) aus Stufe (g) zur Methanol-Syntheseeinheit (B) der Stufe (b) zurückzuführen.

Da Strom (XIV) üblicherweise bei einem Druck von 0,1 bis 0,3 MPa abs vorliegt, die Methanol-synthese jedoch bei einem Druck von 5 bis 10 MPa abs erfolgt, ist es im Falle der Rückführung von Strom (XIV) zur Methanol-Syntheseeinheit (B) vorteilhaft, Strom (XIV) dem Synthesegas-Kompressor zuzuführen und anschließend zusammen mit dem verdichteten Synthesegas in den Reaktor einzuspeisen.

Da der mit Kohlendioxid angereicherte Strom (XIV) in der Regel noch geringe Mengen an Sauerstoff enthält, üblicherweise in der Größenordnung von wenigen Vol.-ppm bis einigen hundert Vol.-ppm, und Sauerstoff die Methanolsynthese hinsichtlich Katalysatorstandzeit und Performance negativ beeinflusst, ist es vorteilhaft, den Sauerstoffgehalt von Strom (XIV) zu verringern. Grundsätzlich können hierfür verschiedene Verfahren eingesetzt werden, die in der Lage sind, geringe Mengen an Sauerstoff aus einem kohlendioxidhaltigen Strom zu entfernen. Ein einfaches Verfahren hierzu stellt die katalytische Hydrierung des Sauerstoffs zu Wasser dar. Als geeignete Katalysatoren hierfür seien exemplarisch Kupfer- oder Edelmetall-haltige Festbettkatalysatoren genannt. Geeignete Verfahren, deren Auslegung und Betrieb sind dem Fachmann bekannt. Somit ist es vorteilhaft, Strom (XIV) vor dessen Rückführung zur Synthesegas-Erzeugungseinheit (A) beziehungsweise zur Methanol-Syntheseeinheit (B) zur Abreicherung des Sauerstoffs katalytisch zu hydrieren. Durch die genannte Hydrierung ist es leicht möglich, den Sauerstoffgehalt auf Werte < 1 Vol.-ppm zu verringern.

Neben dem über Strom (XIV) rückgeführten Kohlendioxid ist es beim erfindungsgemäßen Verfahren auch möglich, auch Kohlendioxid aus anderen Quellen zur Synthesegas-Erzeugungseinheit (A) der Stufe (a) und/oder zur Methanol-Syntheseeinheit (B) der Stufe (b) zuzuführen. Als Kohlendioxid aus sogenannten anderen Quellen sei beispielsweise genannt Kohlendioxid aus diversen Rauchgas, beispielsweise aus dem Rauchgas der Synthesegaserzeugung, aus Kraftwerken oder anderen Verbrennungsprozessen, wobei das Kohlendioxid bevorzugt aus diesen Rauchgasen vorab isoliert wird, beispielsweise durch eine Kohlendioxid-Rückgewinnungseinheit.

Das erfindungsgemäße Verfahren zur Herstellung von Methanol wird kontinuierlich durchgeführt.

Durch die erfindungsgemäßen Verfahrensschritte ist es somit möglich, den Kohlenstoff der werthaltigen Komponenten gezielt für die weitere Synthese vom Methanol wiederzuverwenden, also weiteres Wertprodukt zu erzeugen, und gleichzeitig eine Emission von Kohlendioxid aus der Methanolsynthese zu vermeiden.

Die Methanolsynthese kann dadurch Kohlendioxid-emissionsfrei betrieben werden.

Wie bereits eingangs erwähnt, entspricht eine Stöchiometriezahl S

$$S = \frac{n(H_2) - n(CO_2)}{n(CO) + n(CO_2)} \quad (3)$$

von 2 der rechnerischen Stöchiometrie der Methanolsynthese aus Kohlenmonoxid, Kohlendioxid und Wasserstoff. Abhängig von der Quelle des Synthesegases (II) weist dieses gegebenenfalls bereits eine Stöchiometriezahl S von > 2 auf, etwa bei Synthesegas aus der Dampfreformierung von Methan-haltigem Gas mit einem theoretischen Wert von 3. Im Hinblick auf eine verringerte Bildung an unerwünschten Nebenkomponenten ist jedoch eine Stöchiometriezahl am Reaktoreingang von deutlich oberhalb 2, und besonders bevorzugt von 2,8 bis 3,8 wünschenswert. Um diese Werte zu erreichen, ist ein deutlicher Überschuss an Wasserstoff erforderlich. Allerdings ist festzuhalten, dass Wasserstoff beispielsweise über Strom (IV) aus der Methanol-Syntheseeinheit (B) abgeführt wird. Beim Einsatz eines wasserstoffreichen Synthesegases (II), wie etwa aus der Dampfreformierung kann der Verlust des Wasserstoffs zumeist toleriert werden. Beim Einsatz eines weniger wasserstoffreichen Synthesegases (II) ist es hingegen vielfach von Vorteil, den Wasserstoff aus Strom (IV) nicht in der Verbrennungseinheit (F) zu Wasser zu verbrennen, sondern abzutrennen und der Methanolsynthese zurückzuführen.

In einer bevorzugten Variante des erfindungsgemäßen Verfahrens trennt man daher vor der Zufuhr von Strom (IV) zur Verbrennungseinheit (F) Wasserstoff in einer Wasserstoff-Rückgewinnungseinheit (H) ab und führt diesen zur Methanol-Syntheseeinheit (B) der Stufe (b) zurück. Fig. 3 zeigt ein Blockdiagramm einer allgemeinen Ausführungsform, bei der Wasserstoff in einer Wasserstoff-Rückgewinnungseinheit (H) abgetrennt und rückgeführt wird. Im Allgemeinen ist es vorteilhaft, den abgetrennten Wasserstoff in der Methanol-Syntheseeinheit (B) dem Synthesegas-Kompressor zuzuführen und anschließend zusammen mit dem verdichteten Synthesegas in den Reaktor einzuspeisen.

Grundsätzlich können zur Abtrennung des Wasserstoffs aus Strom (IV) alle Vorrichtungen eingesetzt werden, welche zur Abtrennung von Wasserstoff aus einem Kohlenmonoxid, Kohlendioxid, Wasserstoff und Methan enthaltenden Gasstrom geeignet sind. Entsprechende Vorrichtungen sind dem Fachmann allgemein bekannt, wie beispielsweise die Druckwechseladsorption ("Pressure Swing Adsorption") oder die Permeation. Bevorzugt trennt man beim erfindungsgemäßen Verfahren den Wasserstoff in der Wasserstoff-Rückgewinnungseinheit (H) durch Druckwechseladsorption ab.

Bei der Druckwechseladsorption wird das zu trennende Gas unter einem erhöhten Druck, in der Regel im Bereich von 0,6 bis 1 MPa abs in einen mit einem Adsorbens gefüllten Behälter geleitet. Die Komponenten schwerer als Wasserstoff werden adsorbiert und Wasserstoff als leichte Komponente in aufkonzentrierter Form aus dem Behälter abgeführt. Ist das Adsorberbett weitgehend mit den schwereren Komponenten gesättigt, wird das zu trennende Gas zur weiteren Adsorption in einen anderen, ebenfalls mit Adsorbens gefüllten Behälter geleitet und aus dem vorhergehenden Behälter durch Drucksenkung die schwereren Komponenten durch Desorption wieder freigesetzt und als solche separat entnommen. Hierdurch wird der vorhergehende Behälter wieder regeneriert und für einen neuen Zyklus vorbereitet.

Da zur vollständigen Abtrennung des Wasserstoffs ein besonders hoher Aufwand erforderlich wäre, erfolgt die Abtrennung in der Regel nicht vollständig. Üblicherweise werden beim Einsatz der Wasserstoff-Rückgewinnungseinheit (H) nur 50 bis 95%, bevorzugt $\geq$ 60% sowie bevorzugt $\leq$ 90% des im Gasstrom vorhandenen Wasserstoffs abgetrennt. Daher enthält Strom (IV) auch nach der Abtrennung von Wasserstoff üblicherweise noch weiterhin entsprechende Restmengen an Wasserstoff.

Der abgetrennte Wasserstoff weist im Allgemeinen eine relativ hohe Reinheit auf. Durch die bevorzugte Druckwechseladsorption wird der abgetrennte Wasserstoff in der Regel in einer Reinheit von 90 bis 100 Vol.-%, bevorzugt von $\geq$ 95 Vol.-%, besonders bevorzugt von $\geq$ 99 Vol.-% und ganz besonders bevorzugt von $\geq$ 99,5 Vol.-% erhalten.

Eine Erhöhung des Wasserstoffgehalts am Reaktoreingang und somit der Stöchiometriezahl S am Reaktoreingang ist ferner auch durch Zugabe von weiterem Wasserstoff möglich. Bevorzugt ist daher ein Verfahren, bei dem man der Methanol-Syntheseeinheit (B) in Stufe (b) zusätzlich zu dem über das Synthesegas zugeführten Wasserstoff weiteren Wasserstoff (XVI) zuführt. Fig. 4 zeigt ein Blockdiagramm einer allgemeinen Ausführungsform, bei der zusätzlicher Wasserstoff über Strom (XVI) zugeführt wird.

Bevorzugt wird der weitere Wasserstoff (XVI) dem Synthesegas-Kompressor zugeführt und anschließend zusammen mit dem verdichteten Synthesegas in den Reaktor eingespeist. Falls der weitere Wasserstoff (XVI) jedoch bereits unter einem entsprechend hohen Druck vorliegt, ist es natürlich auch möglich, diesen separat in den Methanol-Synthesereaktor einzuspeisen.

Um die Aufpegelung von reaktionsfremden Stoffen und insbesondere von Inertgasen in der Methanolsynthese zu vermeiden, ist es wünschenswert, als Wasserstoff (XVI) bevorzugt möglichst reinen Wasserstoff oder zumindest Wasserstoff mit einem nur geringen Gehalt an reaktionsfremden Stoffen zuzuführen. Bevorzugt weist der zugeführte Wasserstoff (XVI) einen Wasserstoffgehalt von $\geq$ 80 Vol.-%, besonders bevorzugt von $\geq$ 90 Vol.-%, ganz besonders bevorzugt von $\geq$ 95 Vol.-%, insbesondere von $\geq$ 99 Vol.-% und vor allem von $\geq$ 99,5 Vol.-% auf.

Der zugeführte Wasserstoff (XVI) kann grundsätzlich aus den verschiedensten Quellen stammen. Beispielhaft genannt sei die Zuführung von Wasserstoff aus anderen Produktionsanlagen, in denen Wasserstoff gezielt oder als Nebenprodukt gebildet wird, wie beispielsweise aus Steamcrackern oder Raffinerien, aus der Aufbereitung von Synthesegas, aus der Spaltung von Kohlenwasserstoffen wie zum Beispiel der Pyrolyse von Methan und/oder höheren Kohlenwasserstoffen, oder aus der Elektrolyse von Wasser. Als weitere Beispiele von Produktionsanlagen, in denen Wasserstoff gebildet wird seien genannt die Umsetzung von 1,4-Butandiol zu $\gamma$-Butyrolacton, die Dehydrierung von Propan zu Propen, die Dehydrierung von Methanol zu Formaldehyd, die Dehydrierung von Cyclohexanol zu Cyclohexanon, sowie die Dehydrierung von Cyclododecanol zu Cyclododecanon.

Besonders bevorzugt ist die Verwendung von Wasserstoff aus nachhaltigen Quellen. In diesem Zusammenhang ist vor allem die Elektrolyse von Wasser durch Solar-, Wind- oder Wasserenergie zu nennen.

Die Menge an zuzuführenden Wasserstoff (XVI) richtet sich im Allgemeinen nach der gewünschten Stöchiometriezahl S am Reaktoreingang.

Auch wenn es im Sinne einer energetisch, stofflich und ökologisch verbesserten Herstellung von Methanol bevorzugt ist, zur Erhöhung der Stöchiometriezahl S am Reaktoreingang primär erst einmal den im System vorhandenen Wasserstoff über die Wasserstoff-Rückgewinnungseinheit (H) zu nutzen und erst bei weiterem Bedarf zusätzlichen Wasserstoff über Strom (XVI) zuzuführen, ist es natürlich auch möglich, auch ohne Nutzung einer Wasserstoff-Rückgewinnungseinheit zusätzlichen Wasserstoff über Strom (XVI) zuzuführen. Dies wäre beispielsweise bei einem Überangebot an zusätzlichem Wasserstoff von Interesse und würde die Bereitstellung und den Betrieb einer Wasserstoff-Rückgewinnungseinheit ersparen.

Das Blockdiagramm einer allgemeinen Ausführungsform des erfindungsgemäßen Verfahrens, bei der alle drei Ströme (IV), (V) und (VII) zur Verbrennungseinheit (F) geführt werden, ist in Fig. 1 dargestellt. Die allgemeinen Bedeutungen der Apparateeinheiten und Vorrichtungen (A) bis (G) sowie der Ströme (I) bis (XIV) wurden bereits bei der allgemeinen Beschreibung der Erfindung mit Verweis auf Fig. 1 aufgelistet.

Die nachfolgende Beschreibung bezieht sich auf eine bevorzugte Ausführungsform mit den nachfolgend genannten Charakteristika.

(I) Über Strom (I) wird der Synthesegas-Erzeugungseinheit (A) bevorzugt Methan-haltiges Gas, beispielsweise Erdgas oder Biogas zugeführt.

(A) In der Synthesegas-Erzeugungseinheit (A) wird aus dem bevorzugt Methan-haltigen Gas (I) Synthesegas (II) erzeugt. Bevorzugt hierfür sind die Dampfreformierung, die autotherme Reformierung, eine Kombination aus Dampfreformierung und autothermer Reformierung sowie die partielle Oxidation.

(B) Die Methanol-Syntheseeinheit (B) umfasst einen Kompressor zur Verdichtung des Synthesegases, einen Methanol-Synthesereaktor enthaltend einen Methanol-Synthesekatalysator zur Umsetzung des Synthesegases zu Methanol, einen Kondensator zur Auskondensation von Roh-Methanol und eine entsprechende Leitung zur Rückführung von Synthesekreisgas. Durch Kondensation des erhaltenen Reaktionsgemischs wird Roh-Methanol (III) erhalten und abgeführt. Als Strom (IV) wird nicht-kondensiertes Gas abgeführt.

(C) Die Entspannungseinheit (C) umfasst einen Behälter, in dem das abgetrennte Roh-Methanol (III) entspannt wird und sich sogenanntes Entspannungsgas (V) und entgastes Roh-Methanol (VI) bilden.

(D) Das entgaste Roh-Methanol (VI) wird destillativ aufgearbeitet. In Stufe (d) werden dazu zunächst Leichtsieder (VII) abgetrennt und Methanol im Sumpfstrom (VIII) weiter angereichert. Bei Destillationsvorrichtung (D) handelt es sich üblicherweise um eine Destillationskolonne, auch Leichtsiederkolonne genannt.

(E) Die sogenannte Reindestillation des Methanols erfolgt in Destillationsvorrichtung (E). Aus energetischen Gründen ist hier der Einsatz einer sogenannte Zweidruckdestillation besonders vorteilhaft. Rein-Methanol wird als Strom (X) gewonnen und Schwersieder über Sumpf als Strom (IX) abgetrennt.

(F) Die Verbrennungseinheit (F) umfasst eine Brennkammer. Die abgetrennten Prozessströme (IV), (V) und (VII) werden in diese geleitet und unter Zufuhr eines sauerstoffhaltigen Gases (XI) zu Kohlendioxid verbrannt. Aufgrund der in der Beschreibung genannten Vorteile ist der Einsatz von reinem Sauerstoff als Oxidator besonders bevorzugt.

(G) In der Kohlendioxid-Rückgewinnungseinheit (G) wird aus dem Rauchgas (XII), welches der Verbrennungseinheit (F) entnommen wurde, Kohlendioxid abgetrennt und über Strom (XIV) zur Synthesegas-Erzeugungseinheit (A) und/oder zur Methanol-Syntheseeinheit (B) rückgeführt. Die gestrichelten Linien in Strom (XIV)

versinnbildlichen die genannten Varianten. Im Falle der Rückführung zur Methanol-Syntheseeinheit (B) wird Strom (XIV) bevorzugt dem Synthesegas-Kompressor zugeführt.

Bei der Kohlendioxid-Rückgewinnungseinheit (G) handelt es sich bevorzugt um eine sogenannte Kohlendioxid-Gaswäscheeinheit, in der Kohlendioxid in einem Absorber in einer wässrigen Lösung eines organischen Amins selektiv unter Bildung des Abgasstroms (XIII) ausgewaschen und nachfolgend in einem Desorber wieder freigesetzt wird.

Fig. 2 basiert auf Fig. 1 und unterscheidet sich darin, dass die Verbrennungseinheit (F) neben einer Brennkammer auch einen Kondensator umfasst, in dem Wasser aus dem Brenngas auskondensiert und als Strom (XV) abgeführt wird. Dadurch wird durch ein relativ einfache Maßnahme der Wassergehalt im Rauchgas (XII) deutlich verringert und die nachfolgende Rückgewinnung des Kohlendioxids erleichtert.

Fig. 3 basiert auf Fig. 2 und unterscheidet sich darin, dass vor der Zufuhr von Strom (IV) zur Verbrennungseinheit (F) Wasserstoff in einer Wasserstoff-Rückgewinnungseinheit (H) abgetrennt wird. Die Wasserstoffabtrennung erfolgt bevorzugt durch Druckwechseladsorption. Der abgetrennte Wasserstoff wird als Strom (IVb) zur Methanol-Syntheseeinheit (B) rückgeführt und darin bevorzugt dem Kompressor zugegeben.

Fig. 4 basiert auf Fig. 3 und unterscheidet sich darin, dass über Strom (XVI) der Methanol-Syntheseeinheit (B) zusätzlicher Wasserstoff zugeführt wird.

Fig. 5 basiert auf Fig. 4 und unterscheidet sich darin, dass der mit Kohlendioxid angereicherte Strom (XIV) zur Methanol-Syntheseeinheit (B) zurückgeführt wird.

Das erfindungsgemäße Verfahren ermöglicht die Herstellung von Methanol aus Synthesegas in hoher Ausbeute und Reinheit unter Verwendung der in den Methanol-Syntheseverfahren üblichen Apparate und Verschaltungen, jedoch mit dem entscheidenden Vorteil einer nahezu vollständigen stofflichen Nutzung der kohlenstoffhaltigen Wertkomponenten zur Methanolsynthese und unter Vermeidung einer Emission von Kohlendioxid. Durch die nahezu vollständige stoffliche Nutzung der kohlenstoffhaltigen Wertkomponenten zur Methanolsynthese wird jedoch nicht nur die Emission des klimaschädlichen Kohlendioxids vermieden, sondern auch die Ausbeute an Methanol erhöht. Aufgrund der Verwendung der in der Methanol-Syntheseverfahren üblichen Apparate und Verschaltungen für die eigentliche Methanolsynthese und Methanol-Aufarbeitung lässt sich das erfindungsgemäße Verfahren auch problemlos in bestehenden Methanol-Syntheseanlagen nachrüsten. Technische Eingriffe, Umbauten oder Ergänzungen bei der Synthesegaserzeugung sind auch bei einer Rückführung des abgetrennten Kohlendioxids zur Synthesegaserzeugung aufgrund der absoluten Mengen in der Regel nicht erforderlich. Die erforderlichen Apparate und Verfahrensstufen zur Durchführung des erfindungsgemäßen Verfahrens, sei es für eine Nachrüstung bestehender Methanol-Syntheseanlagen oder dem Neubau von Methanol-Syntheseanlagen, können vom Fachmann mit dem üblichen Fachwissen leicht ausgelegt und installiert werden. Der Aufwand zur Installation und zum Betrieb der erforderlichen Komponenten ist relativ gering. Das erfindungsgemäße Verfahren löst zugleich auch sehr elegant die Ausschleusung unerwünschter Inertgase über den Abgasstrom der Kohlendioxid-Rückgewinnungseinheit. Durch Zufuhr von klimaneutral gewonnenen Wasserstoff, welcher beispielsweise durch Elektrolyse von Wasser auf Basis von Solar-, Wind- oder Wasserenergie gewonnen wurde, besteht zudem die Möglichkeit, die Nachhaltigkeit des erfindungsgemäßen Verfahren weiter zu steigern.

## Beispiele

### Verschaltung 1 (vergleichende Beispiele)

Fig. 6 zeigt ein vereinfachtes Blockdiagramm einer Verschaltung zur Herstellung von Methanol nach dem Stand der Technik. Darin bedeuten:

(A) Synthesegas-Erzeugungseinheit
(B) Methanol-Syntheseeinheit
(C) Entspannungseinheit (Niederdruckentspannung)
(D) Leichtsieder-Kolonne
(E) Methanol-Reinkolonne
(H) Druckwechseladsorption
(I) Erdgas
(II) Synthesegas
(IVb) Rückgewonnener Wasserstoff aus Druckwechseladsorption (H)
(IV) Abgas aus Druckwechseladsorption (H)
(V) Entspannungsgas aus Entspannungseinheit (C)
(VII) Leichtsiederstrom aus Leichtsieder-Kolonne (D)
(IX) Schwersiederstrom aus Methanol-Reinkolonne (E)
(X) Rein-Methanol

(XVI) Wasserstoff ("Frisch-Wasserstoff")

Zudem weist die Verschaltung folgende weiteren Merkmale auf:

- Die Methanol-Syntheseeinheit (B) umfasst einen Kompressor, einen Reaktor, einen Kondensator und einen Synthesegaskreislauf.
- Die Wasserstoff-Rückgewinnungsrate der Druckwechseladsorption (H) beträgt 83%.
- Der Frisch-Wasserstoff (XVI) enthält > 99,5 Vol.-% Wasserstoff.

Sofern im jeweiligen vergleichenden Beispiel nicht anders vermerkt, wird über die Druckwechseladsorption (H) Wasserstoff zurückgewonnen und über Strom (IVb) zum Methanol-Syntheseeinheit (B) zurückgeführt, sowie über Strom (XVI) Frisch-Wasserstoff zugeführt. Die Ströme (IV), (V) und (VII) werden jeweils aus der Verschaltung ausgeschleust.

Verschaltung 2 (erfindungsgemäße Beispiele)

Fig. 5 zeigt ein vereinfachtes Blockdiagramm einer Verschaltung zur erfindungsgemäßen Herstellung von Methanol. Darin bedeuten:

(A) Synthesegas-Erzeugungseinheit
(B) Methanol-Syntheseeinheit
(C) Entspannungseinheit
(D) Leichtsieder-Kolonne
(E) Methanol-Reinkolonne
(F) Verbrennungseinheit
(G) Kohlendioxid-Rückgewinnungseinheit
(H) Druckwechseladsorption

(I) Erdgas
(II) Synthesegas
(IVb) Rückgewonnener Wasserstoff aus Druckwechseladsorption (H)
(IV) Abgas aus Druckwechseladsorption (H)
(V) Entspannungsgas aus Entspannungseinheit (C)
(VII) Leichtsiederstrom aus Leichtsieder-Kolonne (D)
(IX) Schwersiederstrom aus Methanol-Reinkolonne (E)
(X) Rein-Methanol
(XI) Sauerstoffhaltiges Gas
(XII) Rauchgas
(XIII) Abgas aus Kohlendioxid-Rückgewinnungseinheit (G)
(XIV) Kohlendioxid-angereicherter Strom aus Kohlendioxid-Rückgewinnungseinheit (G)
(XV) Auskondensiertes Wasser aus Verbrennungseinheit (F)
(XVI) Wasserstoff ("Frisch-Wasserstoff")

Zudem weist die Verschaltung folgende weiteren Merkmale auf:

- Die Methanol-Syntheseeinheit (B) umfasst einen Kompressor, einen Reaktor, einen Kondensator und einen Synthesegaskreislauf.
- Die Verbrennungseinheit (F) umfasst eine Brennkammer und einen Kondensator.
- Die Umsetzung der brennbaren Komponenten in der Brennkammer zu Kohlendioxid und Wasser erfolgt zu > 99%.
- Die Kohlendioxid-Rückgewinnungsrate der Kohlendioxid-Rückgewinnungseinheit (G) beträgt > 99%.
- Die Wasserstoff-Rückgewinnungsrate der Druckwechseladsorption (H) beträgt 83%.
- Der Frisch-Wasserstoff (XVI) enthält > 99,5 Vol.-% Wasserstoff.

Die Ströme (IV), (V) und (VII) werden in der Verbrennungseinheit (F) unter Zufuhr von reinem Sauerstoff verbrannt und das erhaltene Rauchgas (XII) der Kohlendioxid-Rückgewinnungseinheit (G) zur Rückgewinnung von Kohlendioxid zugeführt. Rückgewonnenes Kohlendioxid wird als Strom (XIV) zur Methanol-Syntheseeinheit (B) rückgeführt. Das Abgas aus der Kohlendioxid-Rückgewinnungseinheit (G) wird aus der Verschaltung ausgeschleust.

Beispiel 1 (Vergleich)

Das vergleichende Beispiel 1 betrifft die Methanolsynthese aus Synthesegas, welches aus der partiellen Oxidation von Erdgas (I) stammt. Die zugrunde liegenden Verschaltung ist in Fig. 6 dargestellt und als "Verschaltung 1" näher beschrieben. Tabelle 1 zeigt die Zusammensetzung des Synthesegaseses (II), welche einer typischen Zusammensetzung für Synthesegas aus der partiellen Oxidation von Erdgas entspricht. Daraus ergibt sich eine Stöchiometriezahl S von 1,625. Um jedoch am Reaktoreingang eine Stöchiometriezahl S von 3,40 einzustellen, ist es erforderlich, zusätzlich zu dem im Synthesegas (II) vorhandenen Wasserstoff, dem innerhalb der Methanol-Syntheseeinheit (B) über das Synthesekreisgas rückgeführten Wasserstoff und dem aus der Druckwechseladsorption (H) rückgeführten Wasserstoff, noch 250,1 $Nm^3$ Frisch-Wasserstoff (XVI) pro Tonne produziertem Rein-Methanol (Strom (X)) zuzuführen. Der Inertengehalt ($CH_4$, $H_2O$, $N_2$, Ar) am Reaktoreingang beträgt 24,4 Vol.-%. Nach heterogenkatalytischer Umsetzung an einem kupferhaltigen Methanol-Synthesekatalysator bei 235°C und einem Druck von 7,7 MPa abs und der weiteren Aufarbeitung des Reaktionsgemisches gemäß dem vereinfachten Blockdiagramm der Fig. 6 ergeben sich die Ströme (IV), (V) (nach Entspannung auf 0,6 MPa abs bei 40°C) und (VII) mit den in Tabelle 1 genannten Mengen und Zusammensetzungen.

Aufgrund der Ausschleusung der Ströme (IV), (V) und (VII) aus der Verschaltung bleiben diese für die weitere Methanolsynthese ungenutzt. In Verfahren nach dem Stand der Technik werden diese üblicherweise nur einer thermischen Verwertung zugeführt, also nicht stofflich genutzt. Somit sind im vorliegenden vergleichenden Beispiel 1 für die Herstellung einer Tonne Rein-Methanol (Strom (X)) 1983 $Nm^3$ Synthesegas (II) erforderlich.

Beispiel 2 (erfindungsgemäß)

Das erfindungsgemäße Beispiel 2 betrifft ebenfalls die Methanolsynthese aus Synthesegas, welches aus der partiellen Oxidation von Erdgas (I) stammt. Die zugrunde liegende Verschaltung ist in Fig. 5 dargestellt und als "Verschaltung 2" näher beschrieben. Tabelle 2 zeigt die Zusammensetzung des Synthesegases (II), welche einer typischen Zusammensetzung für Synthesegas aus der partiellen Oxidation von Erdgas entspricht und identisch zu jener aus Beispiel 1 ist. Somit beträgt auch im erfindungsgemäßen Beispiel 2 die Stöchiometriezahl S des Synthesegases (II) 1,625. Um auch in Beispiel 2 am Reaktoreingang eine Stöchiometriezahl S von 3,40 einzustellen, ist es erforderlich, zusätzlich zu dem im Synthesegas (II) vorhandenen Wasserstoff, dem innerhalb der Methanol-Syntheseeinheit (B) über das Synthesekreisgas rückgeführten Wasserstoff und dem aus der Druckwechseladsorption (H) rückgeführten Wasserstoff, noch 318,0 $Nm^3$ Frisch-Wasserstoff (XVI) pro Tonne produziertem Rein-Methanol (Strom (X)) zuzuführen Der Inertengehalt ($CH_4$, $H_2O$, $N_2$, Ar) am Reaktoreingang beträgt 24,2 Vol.-%. Nach heterogenkatalytischer Umsetzung an einem kupferhaltigen Methanol-Synthesekatalysator bei 235°C und einem Druck von 7,7 MPa abs und der weiteren Aufarbeitung des Reaktionsgemisches gemäß dem vereinfachten Blockdiagramm der Fig. 5 ergeben sich die Ströme (IV), (V) (nach Entspannung auf 0,6 MPa abs bei 40°C) und (VII) mit den in Tabelle 2 genannten Mengen und Zusammensetzungen. Im Gegensatz zum vergleichenden Beispiel 1 werden diese jedoch nicht ungenutzt aus der Verschaltung ausgeschleust, sondern erfindungsgemäß in der Verbrennungseinheit (F) unter Zufuhr von reinem Sauerstoff (XI) verbrannt. Nach Auskondensation von 88,6% des vorhandenen Wassers und dessen Ausschleusung als Strom (XV) wird das kohlendioxidhaltige Rauchgas (XII) einer Kohlendioxid-Rückgewinnungseinheit (G) zugeführt. In dieser werden > 99% des im Rauchgas (XII) enthaltenen Kohlendioxids als Strom (XIV) isoliert und zur Methanol-Syntheseeinheit (B) zurückgeführt.

Für die Herstellung einer Tonne Rein-Methanol (Strom (X)) sind somit im erfindungsgemäßen Beispiel 2 nur 1905 $Nm^3$ Synthesegas (II) erforderlich.

Im Vergleich zum vergleichenden Beispiel 1, bei dem die Ströme (IV), (V) und (VII) ungenutzt aus der Verschaltung ausgeschleust werden, ermöglicht das erfindungsgemäße Verfahren in Beispiel 2 die weitgehende Nutzung der in diesen Strömen enthaltenen Wertkomponenten Kohlenmonoxid, Kohlendioxid, Methan und Dimethylether für die weitere Synthese von Methanol. Konkret führt dies zwar zu einem zusätzlichen Verbrauch von 67,9 $Nm^3$ Frisch-Wasserstoff pro Tonne Rein-Methanol (Strom (X)), erspart jedoch andererseits den Einsatz von 78 $Nm^3$ Synthesegas (II) pro Tonne Rein-Methanol (Strom (X)) und somit der adäquaten Menge an Erdgas, sowie die Emission von zusätzlichem Kohlendioxid, welches ansonsten über eine rein thermische Verwertung der Ströme (IV), (V) und (VII) bei der Verschaltung nach dem Stand der Technik gebildet worden wäre.

Durch die erfindungsgemäße Abtrennung und Rückführung des Kohlendioxids über Strom (XIV) erhöht sich der Kohlendioxidgehalt am Reaktoreingang von 3,0 Vol.-% im vergleichende Beispiel 1 auf 3,9 Vol.-% im erfindungsgemäßen Beispiel 2, liegt aber dennoch weiterhin in einem Bereich, für den die üblicherweise verwendeten Methanol-Synthesekatalysatoren ausgelegt sind.

Beispiel 3 (Vergleich)

Das vergleichende Beispiel 3 betrifft die Methanolsynthese aus Synthesegas, welches aus der autothermen Reformierung von Erdgas stammt. Die zugrunde liegenden Verschaltung ist in Fig. 6 dargestellt und als "Verschaltung 1" näher beschrieben. Tabelle 3 zeigt die Zusammensetzung des Synthesegaseses (II), welche einer typischen Zusammensetzung für Synthesegas aus der autothermen Reformierung von Erdgas entspricht. Daraus ergibt sich eine Stöchiometriezahl S von 1,765. Um jedoch am Reaktoreingang eine Stöchiometriezahl S von 3,40 einzustellen, ist es erforderlich, zusätzlich zu dem im Synthesegas (II) vorhandenen Wasserstoff, dem innerhalb der Methanol-Syntheseeinheit (B) über das Synthesekreisgas rückgeführten Wasserstoff und dem aus der Druckwechseladsorption (H) rückgeführten Wasserstoff, noch 128,7 $Nm^3$ Frisch-Wasserstoff (XVI) pro Tonne produziertem Rein-Methanol (Strom (X)) zuzuführen. Der Inertengehalt ($CH_4$, $H_2O$, $N_2$, Ar) am Reaktoreingang beträgt 24,8 Vol.-%. Nach heterogenkatalytischer Umsetzung an einem kupferhaltigen Methanol-Synthesekatalysator bei 235°C und einem Druck von 7,7 MPa abs und der weiteren Aufarbeitung des Reaktionsgemisches gemäß dem vereinfachten Blockdiagramm der Fig. 6 ergeben sich die Ströme (IV), (V) (nach Entspannung auf 0,6 MPa abs bei 40°C) und (VII) mit den in Tabelle 3 genannten Mengen und Zusammensetzungen.

Aufgrund der Ausschleusung der Ströme (IV), (V) und (VII) aus der Verschaltung bleiben diese für die weitere Methanolsynthese ungenutzt. In Verfahren nach dem Stand der Technik werden diese üblicherweise nur einer thermischen Verwertung zugeführt, also nicht stofflich genutzt.

Somit sind im vorliegenden vergleichenden Beispiel 3 für die Herstellung einer Tonne Rein-Methanol (Strom (X)) 2255 $Nm^3$ Synthesegas (II) erforderlich.

Beispiel 4 (erfindungsgemäß)

Das erfindungsgemäße Beispiel 4 betrifft ebenfalls die Methanolsynthese aus Synthesegas, welches aus der autothermen Reformierung von Erdgas stammt. Die zugrunde liegenden Verschaltung ist in Fig. 5 dargestellt und als "Verschaltung 2" näher beschrieben. Tabelle 4 zeigt die Zusammensetzung des Synthesegases (II), welche einer typischen Zusammensetzung für Synthesegas aus der autothermen Reformierung von Erdgas entspricht und identisch zu jener aus Beispiel 3 ist. Somit beträgt auch im erfindungsgemäßen Beispiel 4 die Stöchiometriezahl S des Synthesegases (II) 1,765. Um auch in Beispiel 4 am Reaktoreingang eine Stöchiometriezahl S von 3,40 einzustellen, ist es erforderlich, zusätzlich zu dem im Synthesegas (II) vorhandenen Wasserstoff, dem innerhalb der Methanol-Syntheseeinheit (B) über das Synthesekreisgas rückgeführten Wasserstoff und dem aus der Druckwechseladsorption (H) rückgeführten Wasserstoff, noch 273,1 $Nm^3$ Frisch-Wasserstoff (XVI) pro Tonne produziertem Rein-Methanol (Strom (X)) zuzuführen. Der Inertengehalt ($CH_4$, $H_2O$, $N_2$, Ar) am Reaktoreingang beträgt 23,7 Vol.-%. Nach heterogenkatalytischer Umsetzung an einem kupferhaltigen Methanol-Synthesekatalysator bei 235°C und einem Druck von 7,7 MPa abs und der weiteren Aufarbeitung des Reaktionsgemisches gemäß dem vereinfachten Blockdiagramm der Fig. 5 ergeben sich die Ströme (IV), (V) (nach Entspannung auf 0,6 MPa abs bei 40°C) und (VII) mit den in Tabelle 4 genannten Mengen und Zusammensetzungen. Im Gegensatz zum vergleichenden Beispiel 3 werden diese jedoch nicht ungenutzt aus der Verschaltung ausgeschleust, sondern erfindungsgemäß in der Verbrennungseinheit (F) unter Zufuhr von reinem Sauerstoff (XI) verbrannt. Nach Auskondensation von 93,5% des vorhandenen Wassers und dessen Ausschleusung als Strom (XV), wird das kohlendioxidhaltige Rauchgas (XII) einer Kohlendioxid-Rückgewinnungseinheit (G) zugeführt. In dieser werden > 99% des im Rauchgas (XII) enthaltenen Kohlendioxids als Strom (XIV) isoliert und zur Methanol-Syntheseeinheit (B) zurückgeführt.

Für die Herstellung einer Tonne Rein-Methanol (Strom (X)) sind somit im erfindungsgemäßen Beispiel 4 nur 2093 $Nm^3$ Synthesegas (II) erforderlich.

Im Vergleich zum vergleichenden Beispiel 3, bei dem die Ströme (IV), (V) und (VII) ungenutzt aus der Verschaltung ausgeschleust werden, ermöglicht das erfindungsgemäße Verfahren in Beispiel 4 die weitgehende Nutzung der in diesen Strömen enthaltenen Wertkomponenten Kohlenmonoxid, Kohlendioxid, Methan und Dimethylether für die weitere Synthese von Methanol. Konkret führt dies zwar zu einem zusätzlichen Verbrauch von 144,4 $Nm^3$ Frisch-Wasserstoff pro Tonne Rein-Methanol (Strom (X)), erspart jedoch andererseits den Einsatz von 162 $Nm^3$ Synthesegas (II) pro Tonne Rein-Methanol (Strom (X)) und somit der adäquaten Menge an Erdgas, sowie die Emission von zusätzlichem Kohlendioxid, welches ansonsten über eine rein thermische Verwertung der Ströme (IV), (V) und (VII) bei der Verschaltung nach dem Stand der Technik gebildet worden wäre.

Durch die erfindungsgemäße Abtrennung und Rückführung des Kohlendioxids über Strom (XIV) erhöht sich der Kohlendioxidgehalt am Reaktoreingang von 6,6 Vol.-% im vergleichenden Beispiel 3 auf 7,4 Vol.-% im erfindungsgemäßen Beispiel 4, liegt aber dennoch weiterhin in einem Bereich, für den die üblicherweise verwendeten Methanol-Synthesekatalysatoren ausgelegt sind.

Beispiel 5 (Vergleich)

Das vergleichende Beispiel 5 betrifft die Methanolsynthese aus Synthesegas, welches aus einer Kombination von Dampfreformierung und autothermer Reformierung von Erdgas stammt. Tabelle 5 zeigt die Zusammensetzung des Synthesegaseses (II), welche einer typischen Zusammensetzung für Synthesegas aus kombinierter Dampf- und autothermer Reformierung von Erdgas entspricht. Daraus ergibt sich eine Stöchiometriezahl S von 2,007. Da der im Synthesegaseses (II) vorhandene Wasserstoff zusammen mit dem über das Synthesekreisgas rückgeführten Wasserstoff bereits ausreicht, um am Reaktoreingang eine Stöchiometriezahl S von 3,40 einzustellen, ist im vorliegenden vergleichenden Beispiel 5 weder eine Rückgewinnung von Wasserstoff durch eine Wasserstoff-Rückgewinnungseinheit mit Rückführung des rückgewonnenen Wasserstoffs, noch die Zufuhr von Frisch-Wasserstoff erforderlich. Die dem vergleichenden Beispiel 5 zugrunde liegende Verschaltung basiert daher zwar ebenfalls auf der in Fig. 6 dargestellten und als "Verschaltung 1" näher beschrieben Verschaltung, weist jedoch weder eine Wasserstoff-Rückgewinnungseinheit (H) mit nachfolgender Rückführung des Wasserstoffs, noch eine Zufuhr von Frisch-Wasserstoff (XVI) auf. Der Inertengehalt ($CH_4$, $H_2O$, $N_2$, Ar) am Reaktoreingang beträgt 24,4 Vol.-%. Nach heterogenkatalytischer Umsetzung an einem kupferhaltigen Methanol-Synthesekatalysator bei 235°C und einem Druck von 7,7 MPa abs und der weiteren Aufarbeitung des Reaktionsgemisches gemäß dem vereinfachten Blockdiagramm der Fig. 6 ergeben sich die Ströme (IV), (V) (nach Entspannung auf 0,6 MPa abs bei 40°C) und (VII) mit den in Tabelle 5 genannten Mengen und Zusammensetzungen.

Aufgrund der Ausschleusung der Ströme (IV), (V) und (VII) aus der Verschaltung bleiben diese für die weitere Methanolsynthese ungenutzt. In Verfahren nach dem Stand der Technik werden diese üblicherweise nur einer thermischen Verwertung zugeführt, also nicht stofflich genutzt. Somit sind im vorliegenden vergleichenden Beispiel 5 für die Herstellung einer Tonne Rein-Methanol (Strom (X)) 2478 $Nm^3$ Synthesegas (II) erforderlich.

Beispiel 6 (erfindungsgemäß)

Das erfindungsgemäße Beispiel 6 betrifft ebenfalls die Methanolsynthese aus Synthesegas, welches aus einer Kombination von Dampfreformierung und autothermer Reformierung von Erdgas stammt. Die zugrunde liegenden Verschaltung ist in Fig. 5 dargestellt und als "Verschaltung 2" näher beschrieben. Tabelle 6 zeigt die Zusammensetzung des Synthesegases (II), welche einer typischen Zusammensetzung für Synthesegas aus kombinierter Dampf- und autothermer Reformierung von Erdgas entspricht und identisch zu jener aus Beispiel 5 ist. Somit beträgt auch im erfindungsgemäßen Beispiel 6 die Stöchiometriezahl S des Synthesegases (II) 2,007. Anders als im vergleichenden Beispiel 5 ist es im erfindungsgemäßen Beispiel 6 zur Einstellung einer Stöchiometriezahl S von 3,40 am Reaktoreingang jedoch erforderlich, zusätzlich zu dem im Synthesegas (II) vorhandenen Wasserstoff und dem innerhalb der Methanol-Syntheseeinheit (B) über das Synthesekreisgas rückgeführten Wasserstoff noch Wasserstoff aus der Druckwechseladsorption (H) sowie 156,1 $Nm^3$ Frisch-Wasserstoff (XVI) pro Tonne produziertem Rein-Methanol (Strom (X)) zuzuführen. Der Inertengehalt ($CH_4$, $H_2O$, $N_2$, Ar) am Reaktoreingang beträgt 24,9 Vol.-%. Nach heterogenkatalytischer Umsetzung an einem kupferhaltigen Methanol-Synthesekatalysator bei 235°C und einem Druck von 7,7 MPa abs und der weiteren Aufarbeitung des Reaktionsgemisches gemäß dem vereinfachten Blockdiagramm der Fig. 5 ergeben sich die Ströme (IV), (V) (nach Entspannung auf 0,6 MPa abs bei 40°C) und (VII) mit den in Tabelle 6 genannten Mengen und Zusammensetzungen. Im Gegensatz zum vergleichenden Beispiel 5 werden diese jedoch nicht ungenutzt aus der Verschaltung ausgeschleust, sondern erfindungsgemäß in der Verbrennungseinheit (F) unter Zufuhr von reinem Sauerstoff (XI) verbrannt. Nach Auskondensation von 94,2% des vorhandenen Wassers und dessen Ausschleusung als Strom (XV), wird das kohlendioxidhaltige Rauchgas (XII) einer Kohlendioxid-Rückgewinnungseinheit (G) zugeführt. In dieser werden > 99% des im Rauchgas (XII) enthaltenen Kohlendioxids als Strom (XIV) isoliert und zur Methanol-Syntheseeinheit (B) zurückgeführt.

Für die Herstellung einer Tonne Rein-Methanol (Strom (X)) sind somit im erfindungsgemäßen Beispiel 6 nur 2238 $Nm^3$ Synthesegas (II) erforderlich.

Beim Einsatz einer Rauchgaswäsche als Kohlendioxid-Rückgewinnungseinheit (G) mit einer Absorptionskolonne und einer Desorptionskolonne, welche über einen Kreuzwärmetauscher miteinander verbunden sind, und welche mit einem aminischen Absorptionsmittel als Absorptionsflüssigkeit betrieben wird, ist für die genannte Kohlendioxid-Rückgewinnung von > 99% ein Absorptionsmittel-Umlaufstrom von 2,71 t Absorptionsmittel pro Tonne Rein-Methanol (Strom (X)) sowie eine Regenerationsenergie zur Regeneration des mit Kohlendioxid beladenen Absorptionsmittels von 0,087 MW pro Tonne Rein-Methanol (Strom (X)) erforderlich.

Im Vergleich zum vergleichenden Beispiel 5, bei dem die Ströme (IV), (V) und (VII) ungenutzt aus der Verschaltung ausgeschleust werden, ermöglicht das erfindungsgemäße Verfahren in Beispiel 6 die weitgehende Nutzung der in diesen Strömen enthaltenen Wertkomponenten Kohlenmonoxid, Kohlendioxid, Methan und Dimethylether für die weitere Synthese von Methanol. Konkret führt dies zwar zu einem zusätzlichen Verbrauch von 156,1 $Nm^3$ Frisch-Wasserstoff pro Tonne Rein-Methanol (Strom (X)), erspart jedoch andererseits den Einsatz von 240 $Nm^3$ Synthesegas (II) pro Tonne Rein-Methanol (Strom (X)) und somit der adäquaten Menge an Erdgas, sowie die Emission von zusätzlichem Kohlendioxid, welches ansonsten über eine rein thermische Verwertung der Ströme (IV), (V) und (VII) bei der Verschaltung nach dem Stand der Technik gebildet worden wäre.

Durch die erfindungsgemäße Abtrennung und Rückführung des Kohlendioxids über Strom (XIV) erhöht sich der Kohlendioxidgehalt am Reaktoreingang von 6,8 Vol.-% im vergleichenden Beispiel 5 auf 7,7 Vol.-% im erfindungsgemäßen Beispiel 6, liegt aber dennoch weiterhin in einem Bereich, für den die üblicherweise verwendeten Methanol-Synthesekatalysatoren ausgelegt sind.

Beispiel 7 (Vergleich)

Das vergleichende Beispiel 7 entspricht dem erfindungsgemäßen Beispiel 6, unterscheidet sich jedoch darin, dass die Ströme (IV), (V) und (VII) in der Verbrennungseinheit (F) unter Zufuhr von Luft (Strom (XI)) anstelle von reinem Sauerstoff verbrannt werden und anschließend 61,2% des vorhandenen Wassers auskondensiert werden. Dadurch erhöht sich die gebildete Menge an Rauchgas (XII) von rund 92 $Nm^3$ (Beispiel 7) signifikant auf rund 649 $Nm^3$ pro Tonne Rein-Methanol (Strom (X)) und der Gehalt an Kohlendioxid im Rauchgas (XII) sinkt deutlich von rund 77 Vol.-% auf nur noch rund 11 Vol.-%. Entsprechend steigt auch die in der Absorptionskolonne erforderliche Absorberfläche auf die etwa 3,8-fache Fläche. Zudem erhöht sich der Absorptionsmittel-Umlaufstrom von 2,71 t auf 4,93 t pro Tonne Rein-Methanol (Strom (X)) und die erforderliche Regenerationsenergie von 0,087 MW auf 0,12 MW pro Tonne Rein-Methanol (Strom (X)). Eine tabellarische Übersicht hierzu findet sich in Tabelle 7.

Durch den Einsatz von reinem Sauerstoff als sauerstoffhaltiges Gas (XI) in der Verbrennungseinheit (F) kann gegenüber dem Einsatz von Luft die Rauchgaswäsche wesentlich effizienter ausgelegt und betrieben werden.

Tabelle 1 Daten zu Beispiel 1 (Vergleich)

| | Synthesegas (II) | FrischWasserstoff (XVI) | (IV) | (V) | (VII) |
|---|---|---|---|---|---|
| | | | | | |
| Menge [$Nm^3$/tMethanol] | 1983 | 250,1 | 16,8 | 10,9 | 10,7 |
| | | | | | |
| CO [Vol.-%] | 33,91 | | 4,50 | 2,77 | 0,11 |
| $CH_3OH$ [Vol.-%] | 0 | | 1,15 | 5,85 | 6,76 |
| $H_2$ [Vol.-%] | 62,20 | > 99,5 | 16,37 | 19,02 | 0,43 |
| $CH_4$ [Vol.-%] | 0,44 | | 27,86 | 30,13 | 7,07 |
| $CH_3OCH_3$ [Vol.-%] | 0 | | 0 | 0,02 | 0,30 |
| $CO_2$ [Vol.-%] | 2,70 | | 6,27 | 21,66 | 44,86 |
| $H_2O$ [Vol.-%] | 0,29 | | 0,04 | 0,14 | 12,11 |
| $N_2$ [Vol.-%] | 0,38 | | 38,13 | 10,22 | 0,42 |
| $O_2$ [Vol.-%] | 0 | | 0 | 0 | 0 |
| Ar [Vol.-%] | 0,08 | | 5,68 | 4,48 | 0,68 |
| | | | | | |
| Stöchiometriezahl S | 1,625 | | | | |

Tabelle 2 Daten zu Beispiel 2 (erfindungsgemäß)

| | Synthesegas (II) | FrischWasserstoff (XVI) | (IV) | (V) | (VII) | (XII) | (XIII) | (XIV) |
|---|---|---|---|---|---|---|---|---|
| | | | | | | | | |
| Menge [$Nm^3$/tMethanol] | 1905 | 318,0 | 16,2 | 11,6 | 11,6 | 43,5 | 12,6 | 30,8 |
| | | | | | | | | |
| CO [Vol.-%] | 33,91 | | 3,87 | 2,27 | 0,08 | 0 | 0 | 0 |

(fortgesetzt)

| | Synthesegas (II) | FrischWasserstoff (XVI) | (IV) | (V) | (VII) | (XII) | (XIII) | (XIV) |
|---|---|---|---|---|---|---|---|---|
| $CH_3OH$ [Vol.-%] | 0 | | 1,09 | 5,68 | 6,05 | 0 | 0 | 0 |
| $H_2$ [Vol.-%] | 62,20 | > 99,5 | 16,69 | 18,15 | 0,36 | 0 | 0 | 0 |
| $CH_4$ [Vol.-%] | 0,44 | | 27,48 | 28,01 | 5,78 | 0 | 0 | 0 |
| $CH_3OCH_3$ [Vol.-%] | 0 | | 0 | 0,02 | 0,28 | 0 | 0 | 0 |
| $CO_2$ [Vol.-%] | 2,70 | | 7,49 | 26,36 | 49,39 | 64,32 | 0,44 | 90,51 |
| $H_2O$ [Vol.-%] | 0,29 | | 0,05 | 0,20 | 12,47 | 7,47 | 2,57 | 9,48 |
| $N_2$ [Vol.-%] | 0,38 | | 37,75 | 9,36 | 0,34 | 17,49 | 60,14 | 0,01 |
| $O_2$ [Vol.-%] | 0 | | 0 | 0 | 0 | 7,21 | 24,77 | 0,004 |
| Ar [Vol.-%] | 0,08 | | 5,59 | 4,17 | 0,56 | 3,51 | 12,08 | 0,001 |
| | | | | | | | | |
| Stöchiometriezahl S | 1,625 | | | | | | | |

Tabelle 3 Daten zu Beispiel 3 (Vergleich)

| | Synthesegas (II) | FrischWasserstoff (XVI) | (IV) | (V) | (VII) |
|---|---|---|---|---|---|
| | | | | | |
| Menge [$Nm^3$/t Methanol] | 2255 | 128,7 | 40,3 | 15,1 | 12,6 |
| | | | | | |
| CO [Vol.-%] | 24,13 | | 3,28 | 1,02 | 0,03 |
| $CH_3OH$ [Vol.-%] | 0 | | 1,04 | 5,10 | 6,87 |
| $H_2$ [Vol.-%] | 65,71 | > 99,5 | 16,99 | 11,98 | 0,18 |
| $CH_4$ [Vol.-%] | 1,22 | | 52,43 | 36,95 | 5,65 |
| $CH_3OCH_3$ [Vol.-%] | 0 | | 0 | 0,03 | 0,24 |
| $CO_2$ [Vol.-%] | 8,36 | | 10,80 | 34,24 | 53,55 |
| $H_2O$ [Vol.-%] | 0,28 | | 0,09 | 0,38 | 12,17 |
| $N_2$ [Vol.-%] | 0,19 | | 9,98 | 1,57 | 0,04 |
| $O_2$ [Vol.-%] | 0 | | 0 | 0 | 0 |
| Ar [Vol.-%] | 0,12 | | 5,39 | 2,86 | 0,30 |
| | | | | | |
| Stöchiometriezahl S | 1,765 | | | | |

Tabelle 4 Daten zu Beispiel 4 (erfindungsgemäß)

| | Synthesegas (II) | FrischWasserstoff (XVI) | (IV) | (V) | (VII) | (XII) | (XIII) | (XIV) |
|---|---|---|---|---|---|---|---|---|
| | | | | | | | | |
| Menge [$Nm^3$/ tMethanol] | 2093 | 273,1 | 38,8 | 14,6 | 13,0 | 70,4 | 12,9 | 57,5 |
| | | | | | | | | |
| CO [Vol.-%] | 24,13 | | 3,18 | 0,89 | 0,02 | 0 | 0 | 0 |

(fortgesetzt)

| | Synthesegas (II) | FrischWasserstoff (XVI) | (IV) | (V) | (VII) | (XII) | (XIII) | (XIV) |
|---|---|---|---|---|---|---|---|---|
| $CH_3OH$ [Vol.-%] | 0 | | 1,02 | 4,91 | 7,38 | 0 | 0 | 0 |
| $H_2$ [Vol.-%] | 65,71 | > 99,5 | 17,76 | 11,79 | 0,16 | 0 | 0 | 0 |
| $CH_4$ [Vol.-%] | 1,22 | | 51,15 | 34,17 | 4,71 | 0 | 0 | 0 |
| $CH_3OCH_3$ [Vol.-%] | 0 | | 0 | 0,03 | 0,23 | 0 | 0 | 0 |
| $CO_2$ [Vol.-%] | 8,36 | | 11,90 | 37,48 | 55,09 | 75,72 | 0,83 | 92,50 |
| $H_2O$ [Vol.-%] | 0,28 | | 0,10 | 0,44 | 11,93 | 6,80 | 3,71 | 7,49 |
| $N_2$ [Vol.-%] | 0,19 | | 9,68 | 1,41 | 0,03 | 6,69 | 36,54 | 0,002 |
| $O_2$ [Vol.-%] | 0 | | 0 | 0 | 0 | 7,11 | 38,82 | 0,003 |
| Ar [Vol.-%] | 0,12 | | 5,21 | 2,65 | 0,26 | 3,68 | 20,09 | 0,001 |
| | | | | | | | | |
| Stöchiometriezahl S | 1,765 | | | | | | | |

Tabelle 5 Daten zu Beispiel 5 (Vergleich)

| | Synthesegas (II) | FrischWasserstoff (XVI) | (IV) | (V) | (VII) |
|---|---|---|---|---|---|
| | | | | | |
| Menge [$Nm^3$/t Methanol] | 2478 | 0 | 119,1 | 15,4 | 12,6 |
| | | | | | |
| CO [Vol.-%] | 21,52 | | 1,76 | 0,95 | 0,02 |
| $CH_3OH$ [Vol.-%] | 0 | | 0,57 | 5,04 | 6,39 |
| $H_2$ [Vol.-%] | 67,94 | | 55,27 | 11,64 | 0,17 |
| $CH_4$ [Vol.-%] | 1,79 | | 31,29 | 38,83 | 5,81 |
| $CH_3OCH_3$ [Vol.-%] | 0 | | 0 | 0,03 | 0,24 |
| $CO_2$ [Vol.-%] | 8,23 | | 6,06 | 34,80 | 53,97 |
| $H_2O$ [Vol.-%] | 0,26 | | 0,05 | 0,40 | 12,38 |
| $N_2$ [Vol.-%] | 0,17 | | 3,45 | 0,95 | 0,02 |
| $O_2$ [Vol.-%] | 0 | | 0 | 0 | 0 |
| Ar [Vol.-%] | 0,09 | | 1,56 | 1,46 | 0,15 |
| | | | | | |
| Stöchiometriezahl S | 2,007 | | | | |

Tabelle 6 Daten zu Beispiel 6 (erfindungsgemäß)

| | Synthesegas (II) | FrischWasserstoff (XVI) | (IV) | (V) | (VII) | (XII) | (XIII) | (XIV) |
|---|---|---|---|---|---|---|---|---|
| | | | | | | | | |
| Menge [$Nm^3$/t Methanol] | 2238 | 156,1 | 58,7 | 15,0 | 12,8 | 92,2 | 15,8 | 76,4 |
| | | | | | | | | |
| CO [Vol.-%] | 21,52 | | 3,09 | 0,77 | 0,02 | 0 | 0 | 0 |

(fortgesetzt)

| | Synthesegas (II) | FrischWasserstoff (XVI) | (IV) | (V) | (VII) | (XII) | (XIII) | (XIV) |
|---|---|---|---|---|---|---|---|---|
| $CH_3OH$ [Vol.-%] | 0 | | 1,01 | 4,81 | 6,96 | 0 | 0 | 0 |
| $H_2$ [Vol.-%] | 67,94 | > 99,5 | 17,07 | 10,76 | 0,14 | 0 | 0 | 0 |
| $CH_4$ [Vol.-%] | 1,79 | | 57,57 | 37,05 | 4,97 | 0 | 0 | 0 |
| $CH_3OCH_3$ [Vol.-%] | 0 | | 0 | 0,03 | 0,23 | 0 | 0 | 0 |
| $CO_2$ [Vol.-%] | 8,23 | | 11,92 | 37,51 | 55,06 | 76,85 | 0,89 | 92,60 |
| $H_2O$ [Vol.-%] | 0,26 | | 0,10 | 0,47 | 12,13 | 6,80 | 3,96 | 7,39 |
| $N_2$ [Vol.-%] | 0,17 | | 6,37 | 0,88 | 0,02 | 5,38 | 31,30 | 0,002 |
| $O_2$ [Vol.-%] | 0 | | 0 | 0 | 0 | 8,66 | 50,41 | 0,004 |
| Ar [Vol.-%] | 0,09 | | 2,87 | 1,42 | 0,14 | 2,31 | 13,44 | 0,001 |
| | | | | | | | | |
| Stöchiometriezahl S | 2,007 | | | | | | | |

Tabelle 7 Vergleich der Beispiele 6 und 7

| | Beispiel 6 (erfindungsgemäß) | Beispiel 7 (Vergleich) |
|---|---|---|
| Strom (XII) [$Nm^3$/t Methanol] | 92 | 649 |
| $CO_2$ [Vol.-%] | 77 | 11 |
| Absorptionsmittel-Umlaufstrom [t Absorptionsflüssigkeit/t Methanol] | 2,71 | 4,93 |
| Regenerationsenergie [MW/t Methanol] | 0,087 | 0,12 |

## Patentansprüche

**1.** Verfahren zur Herstellung von Methanol, bei dem man

(a) aus einem kohlenstoffhaltigen Einsatzstoff (I) in einer Synthesegas-Erzeugungseinheit (A) ein Kohlenmonoxid, Kohlendioxid und Wasserstoff enthaltendes Synthesegas (II) erzeugt,
(b) das Synthesegas (II) aus Stufe (a) einer Methanol-Syntheseeinheit (B) zuführt und bei einer Temperatur von 150 bis 300° C und einem Druck von 5 bis 10 MPa abs in Gegenwart eines Methanol-Synthesekatalysators zu einem Methanol, Wasser, Kohlenmonoxid, Kohlendioxid, Wasserstoff, Dimethylether und Methan haltigen Reaktionsgemisch umsetzt, aus diesem einen mit Methanol und Wasser angereicherten Roh-Methanolstrom (III) auskondensiert, und den Roh-Methanolstrom (III) sowie einen Kohlenmonoxid, Kohlendioxid, Wasserstoff und Methan enthaltenden gasförmigen Strom (IV) aus der Methanol-Syntheseeinheit (B) abführt,
(c) den Roh-Methanolstrom (III) aus Stufe (b) in einer Entspannungseinheit (C) auf einen Druck von 0,1 bis 2 MPa abs entspannt und ein Kohlendioxid und Methan enthaltendes Entspannungsgas (V) sowie einen mit Methanol und Wasser angereicherten entgasten Roh-Methanolstrom (VI) erhält,
(d) aus dem entgasten Roh-Methanolstrom (VI) aus Stufe (c) in einer Destillationsvorrichtung (D) einen Kohlendioxid und Dimethylether enthaltenden Leichtsiederstrom (VII) destillativ abtrennt und einen mit Methanol und Wasser angereicherten Sumpfstrom (VIII) erhält, und
(e) aus dem Sumpfstrom (VIII) aus Stufe (d) in einer weiteren Destillationsvorrichtung (E) einen Wasser enthaltenden Schwersiederstrom (IX) abtrennt und Methanol destillativ als Strom (X) gewinnt,

**dadurch gekennzeichnet, dass** man

(f) die werthaltigen Komponenten Kohlenmonoxid, Kohlendioxid, Dimethylether und Methan der Ströme (IV) sowie von mindestens einem der beiden Ströme (V) und (VII) einer Verbrennungseinheit (F) zuführt und darin unter Zufuhr eines sauerstoffhaltigen Gases (XI), welches einen Sauerstoffgehalt von 30 bis 100 Vol.-% aufweist, verbrennt und kohlendioxidhaltiges Rauchgas (XII) bildet,
(g) aus dem kohlendioxidhaltigen Rauchgas (XII) aus Stufe (f) in einer Kohlendioxid-Rückgewinnungseinheit (G) unter Bildung eines Abgasstroms (XIII) einen mit Kohlendioxid angereicherten Strom (XIV) abtrennt, und
(h) den in der Kohlendioxid-Rückgewinnungseinheit (G) abgetrennten und mit Kohlendioxid angereicherten Strom (XIV) aus Stufe (g) zur Synthesegas-Erzeugungseinheit (A) der Stufe (a) und/oder zur Methanol-Syntheseeinheit (B) der Stufe (b) rückführt.

2. Verfahren nach Anspruch 1, **dadurch gekennzeichnet, dass** man in Stufe (a) als kohlenstoffhaltigen Einsatzstoff (I) Erdgas, Biogas, Kohle, Holz, Kunststoffe, Erdöl, Bionaphtha oder kohlenwasserstoffhaltige Ströme aus der Erdöl- oder Erdgasverarbeitung, aus chemischen Produktionsverfahren, aus nachwachsenden Rohstoffen oder aus dem Kunststoff-Recycling einsetzt.

3. Verfahren nach den Ansprüchen 1 bis 2, **dadurch gekennzeichnet, dass** man in Stufe (a) einen Methan-haltigen Einsatzstoff einsetzt und das Synthesegas (II) durch Dampfreformierung, durch autotherme Reformierung, durch eine Kombination aus Dampfreformierung und autothermer Reformierung oder durch partielle Oxidation erzeugt.

4. Verfahren nach den Ansprüchen 1 bis 3, **dadurch gekennzeichnet, dass** das Synthesegas (II) Methan enthält.

5. Verfahren nach den Ansprüchen 1 bis 4, **dadurch gekennzeichnet, dass** man in der Methanol-Syntheseeinheit (B) in Stufe (b) einen Kupfer und Zink-haltigen Heterogenkatalysator als Methanol-Synthesekatalysator einsetzt.

6. Verfahren nach den Ansprüchen 1 bis 5, **dadurch gekennzeichnet, dass** die Methanol-Syntheseeinheit (B) in Stufe (b) einen Kompressor zur Verdichtung des Synthesegases (II), einen Reaktor zur Umsetzung des Synthesegases (II), einen Kondensator zur Auskondensation des Roh-Methanolstroms (III) und eine Leitung zur Rückführung von nicht auskondensiertem Gas zum Reaktor enthält.

7. Verfahren nach den Ansprüchen 1 bis 6, **dadurch gekennzeichnet, dass** man in Stufe (f) die werthaltigen Komponenten Kohlenmonoxid, Kohlendioxid, Dimethylether und Methan der Ströme (IV), (V) und (VII) der Verbrennungseinheit (F) zuführt.

8. Verfahren nach den Ansprüchen 1 bis 7, **dadurch gekennzeichnet, dass** man der Verbrennungseinheit (F) in Stufe (f) ein sauerstoffhaltiges Gases (XI) mit einem Sauerstoffgehalt von 90 bis 100 Vol.-% zuführt.

9. Verfahren nach den Ansprüchen 1 bis 8, **dadurch gekennzeichnet, dass** die Verbrennungseinheit (F) in Stufe (f) eine Brennkammer und einen Kondensator enthält, man aus dem in der Brennkammer erhaltenen Verbrennungsgas im Kondensator Wasser auskondensiert und als Strom (XV) aus der Verbrennungseinheit (F) abführt, und der verbleibende gasförmige Strom das kohlendioxidhaltige Rauchgases (XII) bildet.

10. Verfahren nach den Ansprüchen 1 bis 9, **dadurch gekennzeichnet, dass** man in der Kohlendioxid-Rückgewinnungseinheit (G) in Stufe (g) Kohlendioxid aus dem kohlendioxidhaltigen Rauchgas (XII) in einem Absorber in einem basischen Lösungsmittel unter Bildung des Abgasstroms (XIII) absorbiert, aus dem mit Kohlendioxid beladenen Lösungsmittel in einem Desorber den mit Kohlendioxid angereicherten Strom (XIV) freigesetzt und das vom Kohlendioxid abgereicherte Lösungsmittel wieder zum Absorber zurückführt.

11. Verfahren nach Anspruch 10, **dadurch gekennzeichnet, dass** man als basisches Lösungsmittel eine wässrige Lösung eines organischen Amins einsetzt.

12. Verfahren nach Anspruch 11, **dadurch gekennzeichnet, dass** man als organisches Amin Monoethanolamin, Piperazin, 2-Amino-2-methyl-1-propanol, Triethylendiamin, N-Methyldiethanolamin oder tert.-Butylaminoethoxyethanol einsetzt.

13. Verfahren nach den Ansprüchen 1 bis 12, **dadurch gekennzeichnet, dass** man in Stufe (h) den Strom (XIV) aus Stufe (g) zur Methanol-Syntheseeinheit (B) der Stufe (b) rückführt.

14. Verfahren nach den Ansprüchen 1 bis 13, **dadurch gekennzeichnet, dass** der mit Kohlendioxid angereicherten

Strom (XIV) aus Stufe (g) Sauerstoff enthält und man Strom (XIV) vor dessen Rückführung zur Synthesegas-Erzeugungseinheit (A) beziehungsweise zur Methanol-Syntheseeinheit (B) zur Abreicherung des Sauerstoffs katalytisch hydriert.

**15.** Verfahren nach den Ansprüchen 1 bis 14, **dadurch gekennzeichnet, dass** man vor der Zufuhr von Strom (IV) zur Verbrennungseinheit (F) Wasserstoff in einer Wasserstoff-Rückgewinnungseinheit (H) abtrennt und diesen zur Methanol-Syntheseeinheit (B) der Stufe (b) rückführt.

**16.** Verfahren nach Anspruch 15, **dadurch gekennzeichnet, dass** man den Wasserstoff in der Wasserstoff-Rückgewinnungseinheit (H) durch Druckwechseladsorption abtrennt.

**17.** Verfahren nach den Ansprüchen 1 bis 16 **dadurch gekennzeichnet, dass** man der Methanol-Syntheseeinheit (B) in Stufe (b) zusätzlich zu dem über das Synthesegas zugeführten Wasserstoff weiteren Wasserstoff (XVI) zuführt.

## Claims

**1.** A process for preparing methanol by

(a) producing a synthesis gas (II) comprising carbon monoxide, carbon dioxide and hydrogen from a carbonaceous feedstock (I) in a synthesis gas production unit (A),
(b) feeding the synthesis gas (II) from stage (a) to a methanol synthesis unit (B) and converting it at a temperature of 150 to 300°C and a pressure of 5 to 10 MPa abs in the presence of a methanol synthesis catalyst to a reaction mixture containing methanol, water, carbon monoxide, carbon dioxide, hydrogen, dimethyl ether and methane, condensing a methanol- and water-enriched crude methanol stream (III) out of said reaction mixture, and conducting the crude methanol stream (III) and a gaseous stream (IV) comprising carbon monoxide, carbon dioxide, hydrogen and methane out of the methanol synthesis unit (B),
(c) expanding the crude methanol stream (III) from stage (b) in an expansion unit (C) to a pressure of 0.1 to 2 MPa abs, and obtaining an expansion gas (V) comprising carbon dioxide and methane and a degassed crude methanol stream (VI) enriched with methanol and water,
(d) separating a carbon dioxide- and dimethyl ether-comprising low boiler stream (VII) by distillation from the degassed crude methanol stream (VI) from stage (c) in a distillation apparatus (D), and obtaining a methanol- and water-enriched bottom stream (VIII), and
(e) separating a water-containing high boiler stream (IX) from the bottom stream (VIII) from stage (d) in a further distillation apparatus (E), and obtaining methanol by distillation as stream (X),

which comprises

(f) feeding the carbon monoxide, carbon dioxide, dimethyl ether and methane components of value in streams (IV) and in at least one of the two streams (V) and (VII) to a combustion unit (F) and combusting them therein with supply of an oxygenous gas (XI) having an oxygen content of 30% to 100% by volume, forming carbon dioxide-containing flue gas (XII),
(g) separating a carbon dioxide-enriched stream (XIV) from the carbon dioxide-containing flue gas (XII) from stage (f) in a carbon dioxide recovery unit (G) to form an offgas stream (XIII), and
(h) recycling the carbon dioxide-enriched stream (XIV) separated off in the carbon dioxide recovery unit (G) from stage (g) to the synthesis gas production unit (A) of stage (a) and/or to the methanol synthesis unit (B) of stage (b).

**2.** The process according to claim 1, wherein the hydrocarbonaceous feedstock (I) used in stage (a) is natural gas, biogas, coal, wood, plastics, mineral oil, bionaphtha or hydrocarbonaceous streams from mineral oil or natural gas processing, from chemical production processes, from renewable raw materials or from plastics recycling.

**3.** The process according to claim 1 or 2, wherein a methane-containing feedstock is used in stage (a) and the synthesis gas (II) is produced by steam reforming, by autothermal reforming, by a combination of steam reforming and autothermal reforming, or by partial oxidation.

**4.** The process according to claims 1 to 3, wherein the synthesis gas (II) comprises methane.

5. The process according to claims 1 to 4, wherein a copper- and zinc-containing heterogeneous catalyst is used as methanol synthesis catalyst in the methanol synthesis unit (B) in stage (b).

6. The process according to claims 1 to 5, wherein the methanol synthesis unit (B) in stage (b) comprises a compressor for compression of the synthesis gas (II), a reactor for conversion of the synthesis gas (II), a condenser for condensing out the crude methanol stream (III), and a conduit for recycling of uncondensed gas to the reactor.

7. The process according to claims 1 to 6, wherein in stage (f), the carbon monoxide, carbon dioxide, dimethyl ether and methane components of value in streams (IV), (V) and (VII) are fed to the combustion unit (F).

8. The process according to claims 1 to 7, wherein the combustion unit (F) in stage (f) is supplied with an oxygenous gas (XI) having an oxygen content of 90% to 100% by volume.

9. The process according to claims 1 to 8, wherein the combustion unit (F) in stage (f) comprises a combustion chamber and a condenser, water is condensed out of the combustion gas obtained in the combustion chamber in the condenser and conducted out of the combustion unit (F) as stream (XV), and the remaining gaseous stream constitutes the carbon dioxide-containing flue gas (XII) .

10. The process according to claims 1 to 9, wherein, in the carbon dioxide recovery unit (G) in stage (g), carbon dioxide is absorbed from the carbon dioxide-containing flue gas (XII) in an absorber in a basic solvent to form the offgas stream (XIII), the carbon dioxide-enriched stream (XIV) is released from the carbon dioxide-laden solvent in a desorber, and the carbon dioxide-depleted solvent is returned to the absorber.

11. The process according to claim 10, wherein the basic solvent used is an aqueous solution of an organic amine.

12. The process according to claim 11, wherein the organic amine used is monoethanolamine, piperazine, 2-amino-2-methyl-1-propanol, triethylenediamine, N-methyldiethanolamine or tert-butylaminoethoxyethanol.

13. The process according to claims 1 to 12, wherein, in stage (h), stream (XIV) from stage (g) is recycled to the methanol synthesis unit (B) of stage (b).

14. The process according to claims 1 to 13, wherein the carbon dioxide-enriched stream (XIV) from stage (g) comprises oxygen, and stream (XIV), before it is recycled to the synthesis gas production unit (A) or to the methanol synthesis unit (B), is catalytically hydrogenated to deplete the oxygen.

15. The process according to claims 1 to 14, wherein, before stream (IV) is fed to the combustion unit (F), hydrogen is separated off in a hydrogen recovery unit (H) and recycled to the methanol synthesis unit (B) of stage (b).

16. The process according to claim 15, wherein the hydrogen is separated off by pressure swing adsorption in the hydrogen recovery unit (H).

17. The process according to claims 1 to 16, wherein the methanol synthesis unit (B) in stage (b) is supplied with further hydrogen (XVI) in addition to the hydrogen supplied via the synthesis gas.

**Revendications**

1. Procédé pour la préparation de méthanol, dans lequel

(a) on produit, à partir d'une matière à traiter (I) contenant du carbone, dans une unité de production de gaz de synthèse (A), un gaz de synthèse (II) contenant du monoxyde de carbone, du dioxyde de carbone et de l'hydrogène,
(b) on introduit le gaz de synthèse (II) de l'étape (a) dans une unité de synthèse de méthanol (B) et on le transforme, à une température de 150 à 300°C et à une pression de 5 à 10 MPa abs en présence d'un catalyseur de synthèse de méthanol en un mélange réactionnel contenant du méthanol, de l'eau, du monoxyde de carbone, du dioxyde de carbone, de l'hydrogène, du diméthyléther et du méthane, on sépare par condensation à partir de celui-ci un flux (III) de méthanol brut enrichi en méthanol et en eau et on évacue le flux (III) de méthanol brut ainsi qu'un flux gazeux (IV), contenant du monoxyde de carbone, du dioxyde de carbone, de l'hydrogène et du

méthane, de l'unité de synthèse de méthanol (B),

(c) on détend le flux (III) de méthanol brut de l'étape (b) dans une unité de détente (C) à une pression de 0,1 à 2 MPa abs et on obtient un gaz de détente (V) contenant du dioxyde de carbone et du méthane ainsi qu'un flux (VI) de méthanol brut dégazé enrichi en méthanol et en eau,

(d) on sépare par distillation, à partir du flux (VI) de méthanol brut dégazé de l'étape (c), dans un dispositif de distillation (D), un flux (VII) de substances à bas point d'ébullition contenant du dioxyde de carbone et du diméthyléther et on obtient un flux de fond (VIII) enrichi en méthanol et en eau et

(e) on sépare, à partir du flux de fond (VIII) de l'étape (d), dans un autre dispositif de distillation (E), un flux (IX) de substances à point d'ébullition élevé contenant de l'eau et on obtient par distillation le méthanol en tant que flux (X),

**caractérisé en ce que**

(f) on introduit les composants de valeur, monoxyde de carbone, dioxyde de carbone, diméthyléther et méthane, du flux (IV) ainsi que d'au moins l'un des deux flux (V) et (VII) dans une unité de combustion (F) et on les brûle, sous apport d'un gaz (XI) contenant de l'oxygène, qui présente une teneur en oxygène de 30 à 100% en volume, et on forme un gaz de fumée (XII) contenant du dioxyde de carbone,

(g) on sépare à partir du gaz de fumée (XII) contenant du dioxyde de carbone de l'étape (f), dans une unité de récupération de dioxyde de carbone (G), avec formation d'un effluent gazeux (XIII), un flux (XIV) enrichi en dioxyde de carbone et

(h) on recycle le flux (XIV) séparé dans l'unité de récupération de dioxyde de carbone (G) et enrichi en dioxyde de carbone de l'étape (g) dans l'unité de production de gaz de synthèse (A) de l'étape (a) et/ou dans l'unité de synthèse de méthanol (B) de l'étape (b).

**2.** Procédé selon la revendication 1, **caractérisé en ce qu'**on utilise, dans l'étape (a), en tant que matière à traiter (I) contenant du carbone, du gaz naturel, du biogaz, du charbon, du bois, des matériaux synthétiques, du pétrole, du bionaphta ou des flux hydrocarbonés provenant du traitement du pétrole ou du gaz naturel, provenant de procédés de production chimiques, provenant de matières premières renouvelables ou provenant du recyclage de matériaux synthétiques.

**3.** Procédé selon les revendications 1 à 2, **caractérisé en ce qu'**on utilise, dans l'étape (a), une matière à traiter contenant du méthane et on produit le gaz de synthèse (II) par reformage à la vapeur, par reformage autothermique, par une combinaison de reformage à la vapeur et de reformage autothermique ou par oxydation partielle.

**4.** Procédé selon les revendications 1 à 3, **caractérisé en ce que** le gaz de synthèse (II) contient du méthane.

**5.** Procédé selon les revendications 1 à 4, **caractérisé en ce qu'**on utilise, dans l'unité de synthèse de méthanol (B) dans l'étape (b), un catalyseur hétérogène contenant du cuivre et du zinc en tant que catalyseur de synthèse de méthanol.

**6.** Procédé selon les revendications 1 à 5, **caractérisé en ce que** l'unité de synthèse de méthanol (B) dans l'étape (b) contient un compresseur pour la compression du gaz de synthèse (II), un réacteur pour la transformation du gaz de synthèse (II), un condenseur pour la séparation par condensation du flux (III) et une conduite pour le recyclage du gaz non séparé par condensation vers le réacteur.

**7.** Procédé selon les revendications 1 à 6, **caractérisé en ce qu'**on introduit, dans l'étape (f), les composants de valeur, monoxyde de carbone, dioxyde de carbone, diméthyléther et méthane, des flux (IV), (V) et (VII), dans l'unité de combustion (F).

**8.** Procédé selon les revendications 1 à 7, **caractérisé en ce que** l'unité de combustion (F) dans l'étape (f) est alimentée en un gaz (XI) contenant de l'oxygène présentant une teneur en oxygène de 90 à 100% en volume.

**9.** Procédé selon les revendications 1 à 8, **caractérisé en ce que** l'unité de combustion (F) dans l'étape (f) contient une chambre de combustion et un condenseur, on sépare par condensation, dans le condenseur, de l'eau à partir du gaz de combustion obtenu dans la chambre de combustion et on l'évacue en tant que flux (XV) de l'unité de combustion (F) et le flux gazeux résiduel forme le gaz de fumée (XII) contenant du dioxyde de carbone.

**10.** Procédé selon les revendications 1 à 9, **caractérisé en ce qu'**on absorbe, dans l'unité de récupération de dioxyde

de carbone (G) dans l'étape (g), le dioxyde de carbone à partir du gaz de fumée (XII) contenant du dioxyde de carbone dans un absorbeur dans un solvant basique avec formation de l'effluent gazeux (XIII), on libère, à partir du solvant chargé de dioxyde de carbone, dans un désorbeur, le flux (XIV) enrichi en dioxyde de carbone et on recycle de nouveau le solvant appauvri en dioxyde de carbone dans l'absorbeur.

**11.** Procédé selon la revendication 10, **caractérisé en ce qu'**on utilise, comme solvant basique, une solution aqueuse d'une amine organique.

**12.** Procédé selon la revendication 11, **caractérisé en ce qu'**on utilise, comme amine organique, de la monoéthanolamine, de la pipérazine, du 2-amino-2-méthyl-1-propanol, de la triéthylènediamine, de la N-méthyldiéthanolamine ou du tert-butylaminoéthoxyéthanol.

**13.** Procédé selon les revendications 1 à 12, **caractérisé en ce qu'**on recycle, dans l'étape (h), le flux (XIV) de l'étape (g) dans l'unité de synthèse de méthanol (B) de l'étape (b).

**14.** Procédé selon les revendications 1 à 13, **caractérisé en ce que** le flux (XIV) enrichi en dioxyde de carbone de l'étape (g) contient de l'oxygène et on soumet le flux (XIV) à une hydrogénation catalytique, en vue de l'appauvrissement en oxygène, avant son recyclage dans l'unité de production de gaz de synthèse (A) ou dans l'unité de synthèse de méthanol (B).

**15.** Procédé selon les revendications 1 à 14, **caractérisé en ce que**, avant l'introduction du flux (IV) dans l'unité de combustion (F), on sépare l'hydrogène dans une unité de récupération d'hydrogène (H) et on recycle celui-ci dans l'unité de synthèse de méthanol (B) de l'étape (b).

**16.** Procédé selon la revendication 15, **caractérisé en ce qu'**on sépare l'hydrogène dans l'unité de récupération d'hydrogène (H) par adsorption modulée en pression.

**17.** Procédé selon les revendications 1 à 16, **caractérisé en ce qu'**on alimente l'unité de synthèse de méthanol (B) dans l'étape (b), en plus de l'hydrogène introduit par l'intermédiaire du gaz de synthèse, en hydrogène (XVI) supplémentaire.

Fig. 1

Blockdiagramm (erfindungsgemäß)

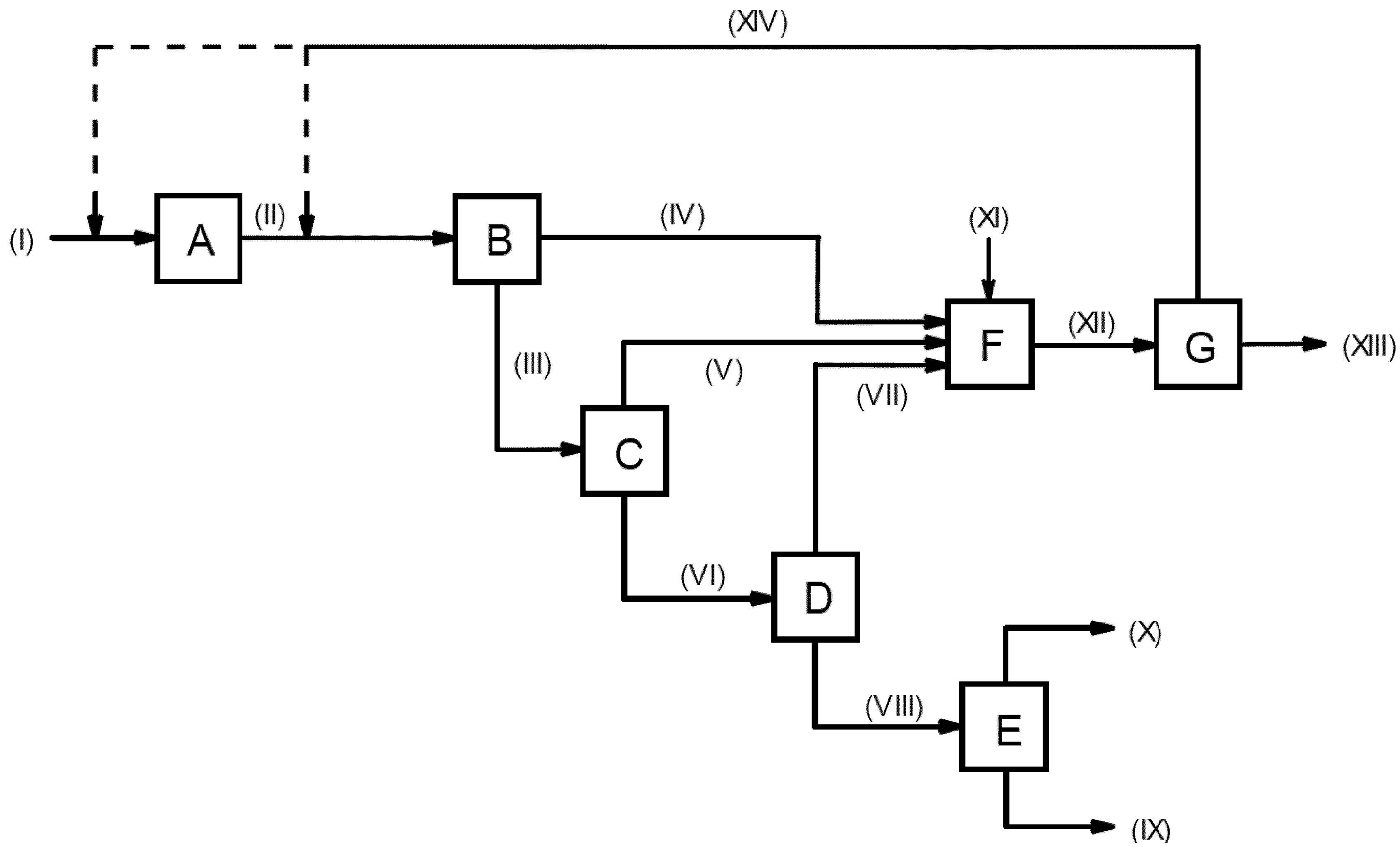

Fig. 2

Blockdiagramm (erfindungsgemäß)

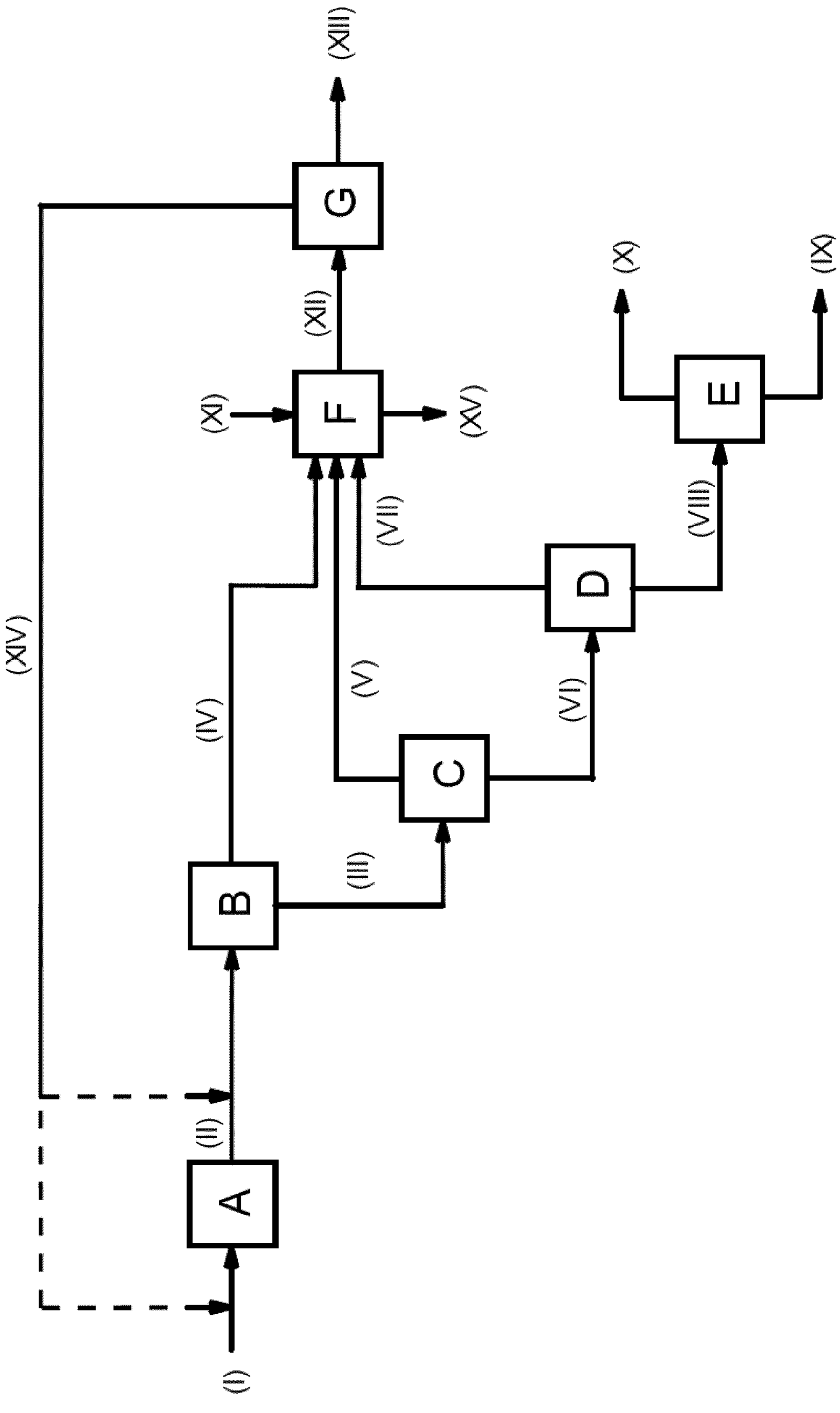

Fig. 3

Blockdiagramm (erfindungsgemäß)

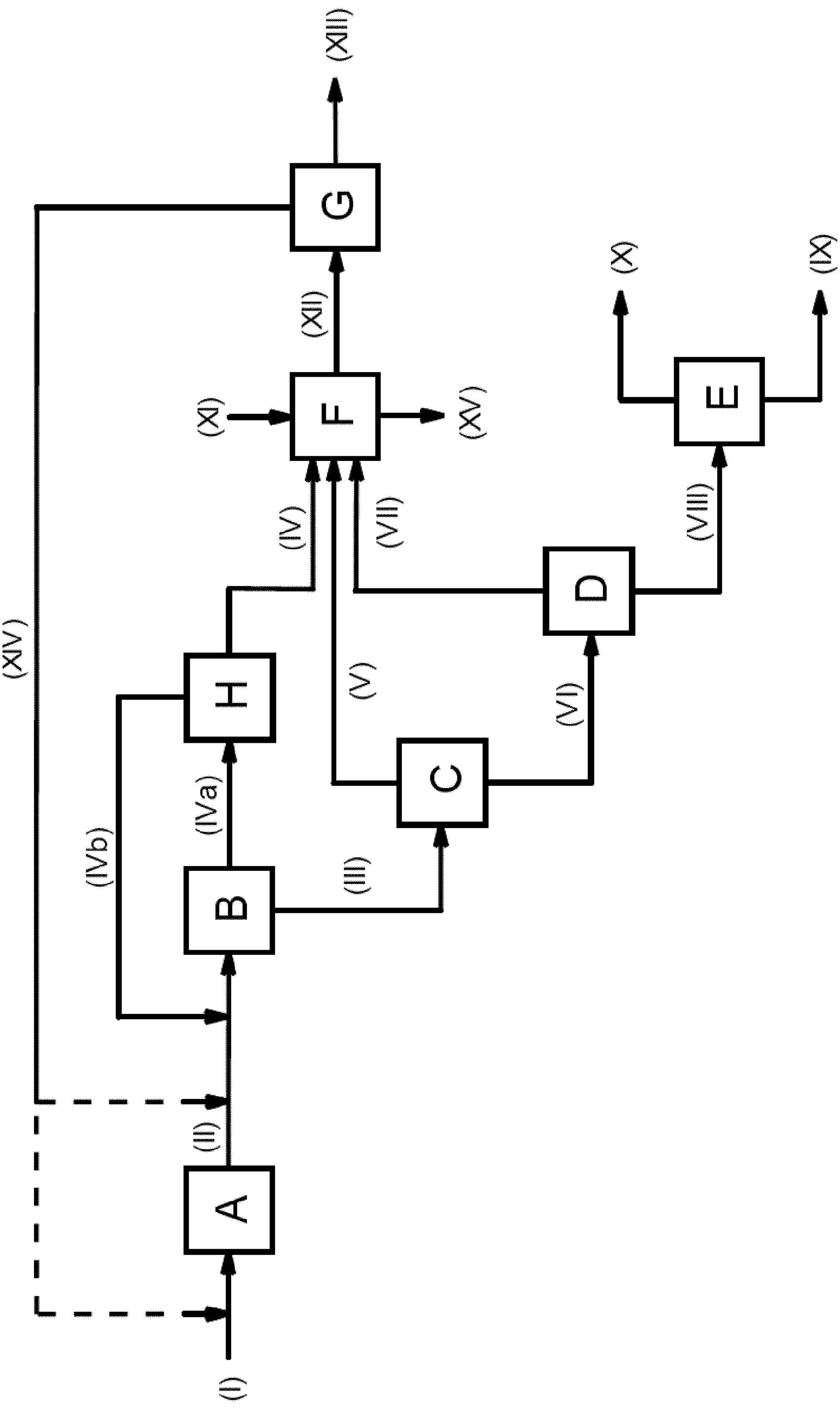

Fig. 4

Blockdiagramm (erfindungsgemäß)

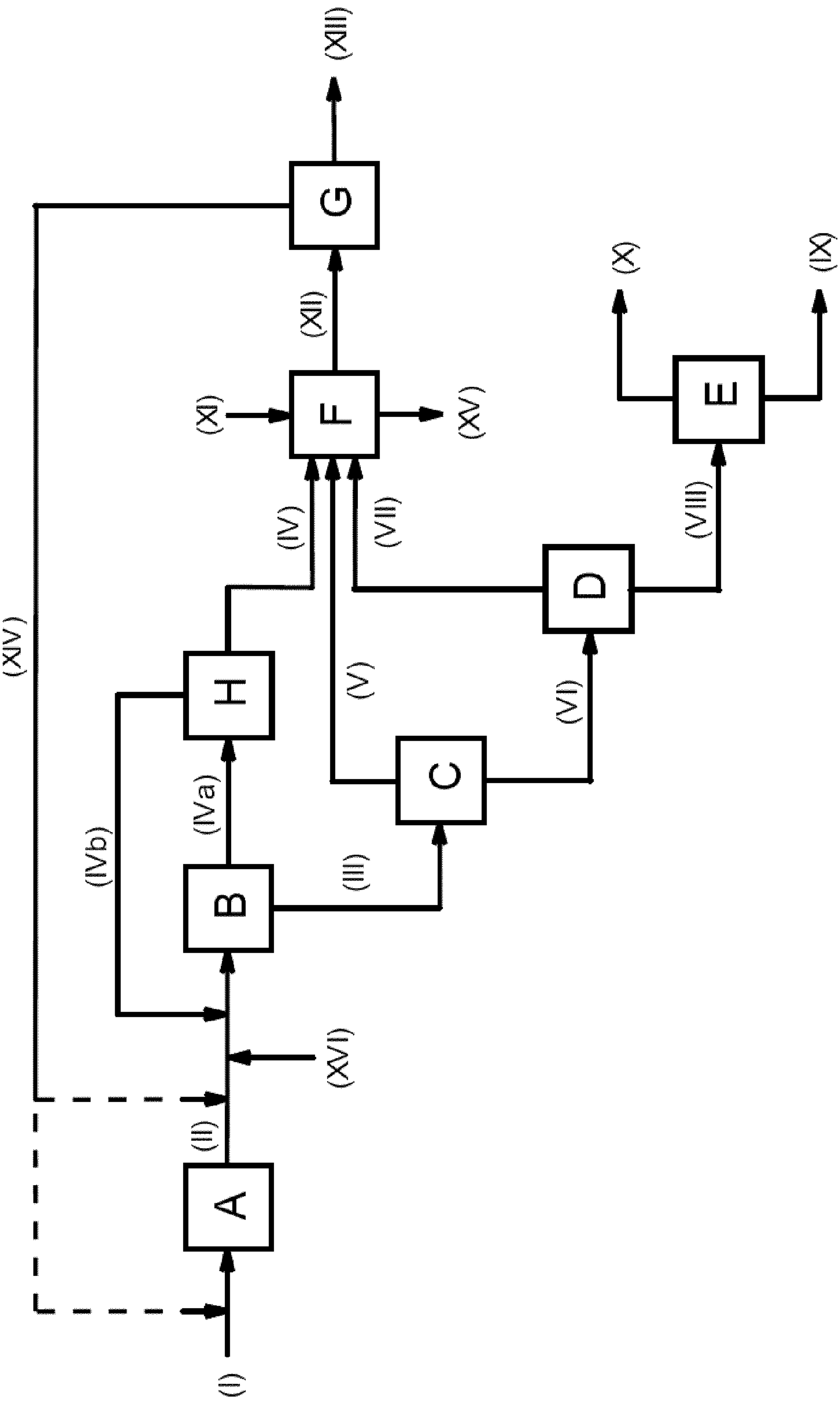

Fig. 5

Blockdiagramm (erfindungsgemäß)

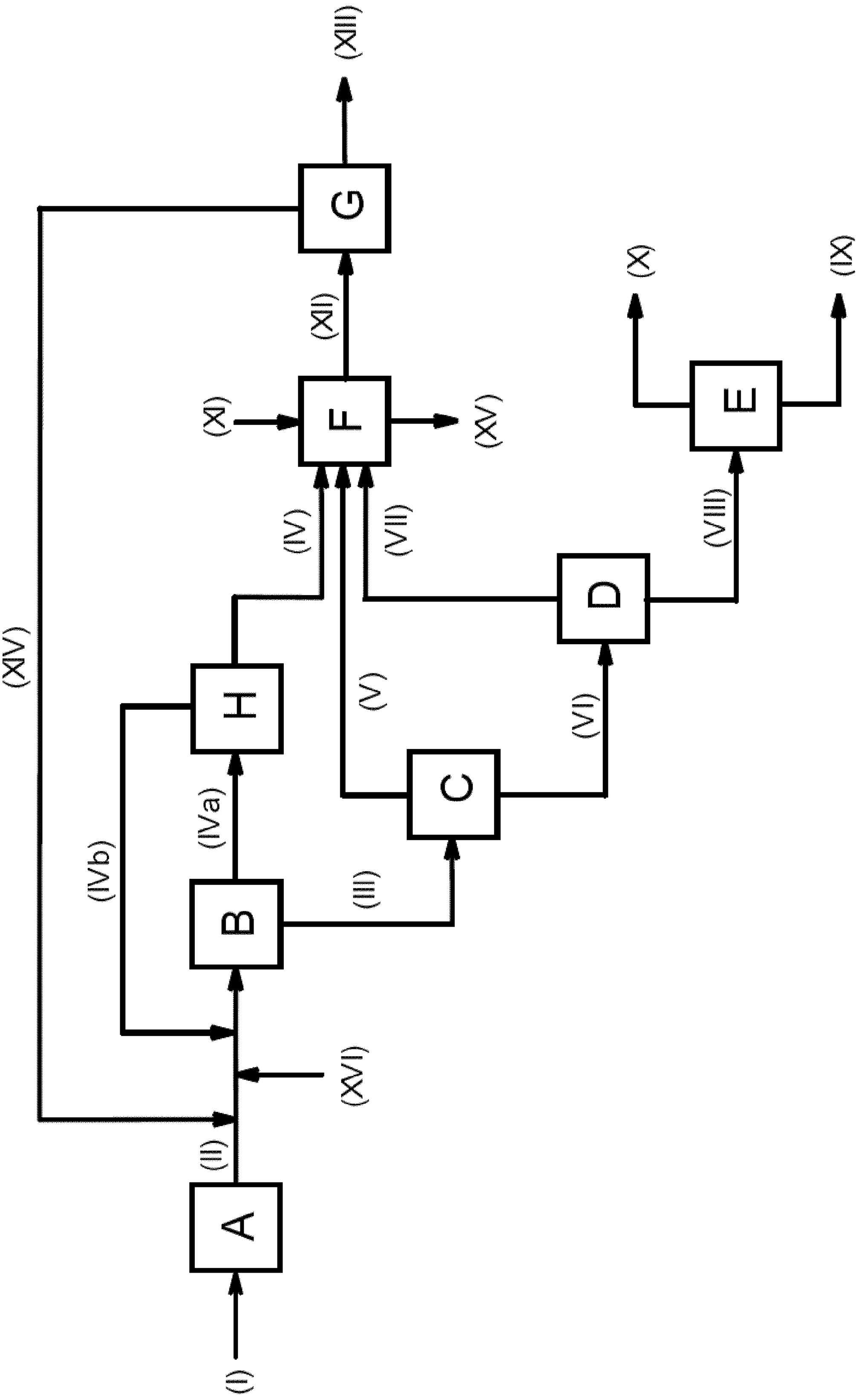

Fig. 6

Blockdiagramm (Vergleich)

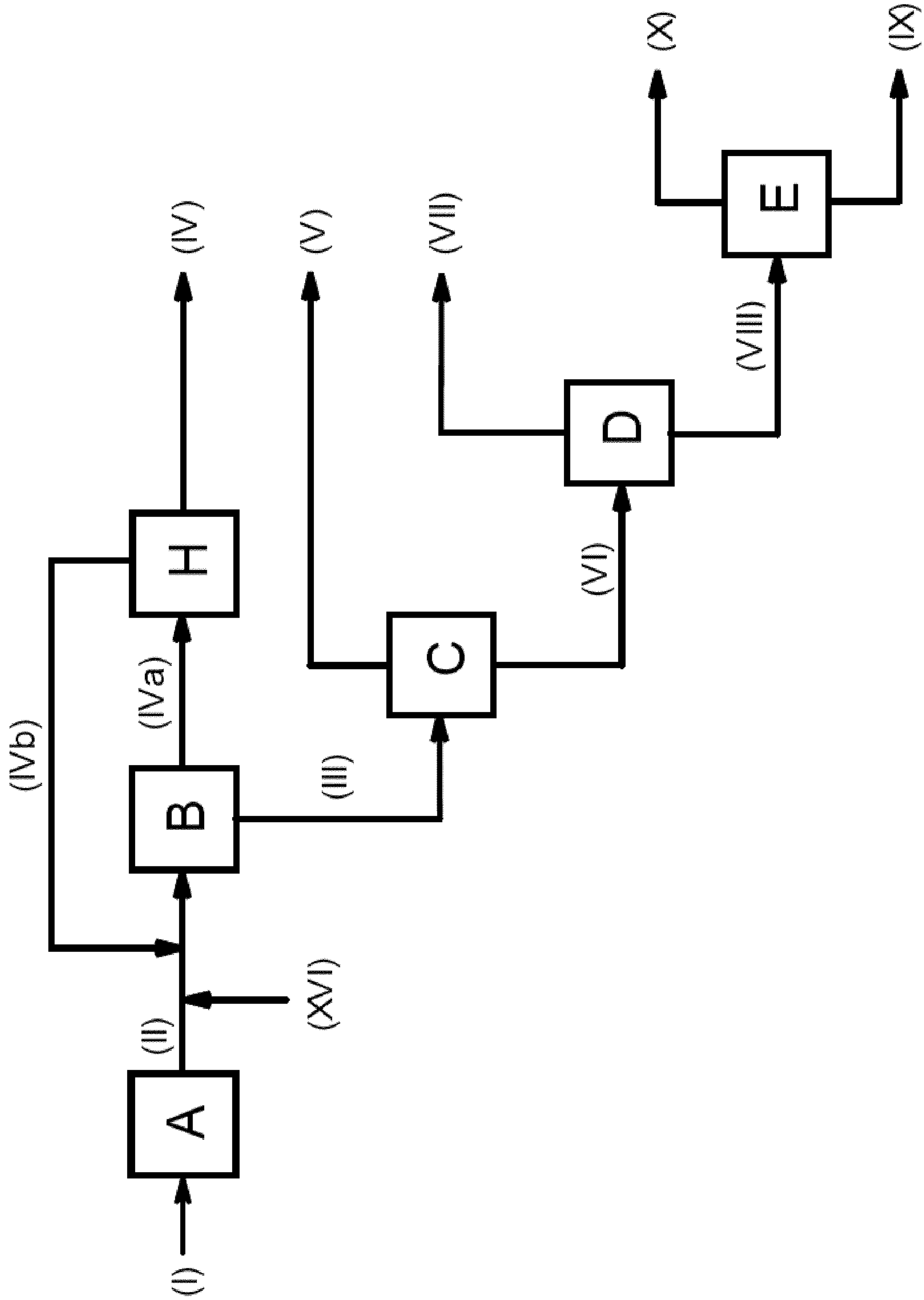

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3178804 A1
- EP 2228358 A1
- US 8829059 B
- US 2008236390 A
- US 2010192770 A
- US 2011094381 A


### In der Beschreibung aufgeführte Nicht-Patentliteratur


- Methanol. Ullmann's Encyclopedia of Industrial Chemistry. Wiley-VCH Verlag GmbH & Co. KGaA, 2012
- **S. REDDY et al.** *Energy Procedia,* 2014, vol. 63, 1407-1414
- Carbon Dioxide. Ullmann's Encyclopedia of Industrial Chemistry. Wiley-VCH Verlag GmbH & Co. KGaA, Weinheim, 2014